# EUROPEAN PATENT SPECIFICATION

(11) **EP 4 171 474 B1**
(45) Date of publication and mention of the grant of the patent: **18.11.2026**
(21) Application number: 21734839.0
(22) Date of filing: 23.06.2021
(51) Int. Cl.: A61K 8/06, A61K 8/73, A61K 8/81, A61K 8/891, A61K 8/898, A61Q 5/02, A61Q 5/12

(54) **COMPOSITION COMPRISING A PARTICULAR SILICONE EMULSION, A PARTICULAR (METH)ACRYLAMIDE POLYMER, SURFACTANTS AND A CATIONIC POLYSACCHARIDE**
ZUSAMMENSETZUNG MIT EINER BESTIMMTEN SILIKONEMULSION, EINEM BESTIMMTEN (METH)ACRYLAMIDPOLYMER, TENSIDEN UND EINEM KATIONISCHEN POLYSACCHARID
COMPOSITION COMPRENANT UNE ÉMULSION DE SILICONE PARTICULIÈRE, UN POLYMÈRE (MÉTH)ACRYLAMIDE PARTICULIER, DES TENSIOACTIFS ET UN POLYSACCHARIDE CATIONIQUE

(30) Priority: 26.06.2020 IN 202021027194; 10.08.2020 FR 2008398
(43) Date of publication of application: 03.05.2023
(73) Proprietor: L'OREAL, 75008 Paris (FR)
(72) Inventor: JOSHI, Sarish, Maharashtra Mumbai 400071 (IN); TULSYAN, Harshada, Maharashtra Mumbai 400071 (IN); ADULKAR, Tejal, Maharashtra Mumbai 400071 (IN)
(74) Representative: Casalonga
(86) International application number: PCT/EP2021/067090
(87) International publication number: WO 2021/259973

(56) References cited:
- WO-A1-00/37041
- WO-A1-00/64410
- WO-A1-2017/108824
- US-B2- 6 908 889

## Description

The present invention relates to a composition for treating keratin fibres, and in particular human keratin fibres such as hair, which comprises a specific oil-in-water-type silicone emulsion, one or more particular (meth)acrylamide polymer, optionally one or more anionic surfactants, optionally one or more amphoteric or zwitterionic surfactants and one or more cationic polysaccharides.

The invention also relates to a process for treating keratin fibres, preferably for washing and/or conditioning keratin fibres, comprising the application onto the keratin fibres of the composition according to the invention.

Finally, the invention relates to the use of such a composition for washing and/or conditioning keratin fibres, preferably the hair.

It is common practice to use detergent cosmetic compositions such as shampoos and shower gels, based essentially on surfactants, for washing keratin materials especially such as the hair and the skin. These compositions are applied to the keratin materials, which are preferably wet, and the lather generated by massaging or rubbing with the hands or a toiletry flannel makes it possible, after rinsing with water, to remove the diverse types of soiling initially present on the hair or the skin.

These compositions contain substantial contents of "detergent" surfactants, which, in order to be able to formulate cosmetic compositions with good washing power, must especially give them good foaming power.

The surfactants that are useful for this purpose are generally of anionic, nonionic and/or amphoteric type, and particularly of anionic type.

These compositions have generally a good washing power, but the intrinsic cosmetic properties associated with them nevertheless remain fairly poor, owing in particular to the fact that the relatively aggressive nature of such a cleaning treatment can, in the long run, lead to more or less pronounced damage to the hair fibre, this damage being associated in particular with the gradual removal of the lipids or proteins contained in or on the surface of this fibre.

Thus, in order to improve the cosmetic properties of the above detergent compositions, and more particularly those which are to be applied to sensitized hair (i.e. hair which has been damaged or made brittle, in particular under the chemical action of atmospheric agents and/or hair treatments such as permanent-waving, dyeing or bleaching), it is now common to introduce additional cosmetic agents known as conditioners into these compositions. These conditioners are intended mainly to repair or limit the harmful or undesirable effects induced by the various treatments or aggressions to which the hair fibres are subjected more or less repeatedly. They may, of course, also improve the cosmetic behaviour of natural hair.

The conditioners most commonly used to date in shampoos include cationic polymers, silicones and/or silicone derivatives, which give washed, dry or wet hair an ease-of disentangling, softness and smoothness which are markedly better than that which can be obtained with corresponding cleaning compositions from which they are absent.

In particular, it is known to use a mixture of silicone and cationic polymer. However, the compositions containing them still have numerous disadvantages, such as leading to an insufficient deposit of silicones on hair and impacting therefore strongly on their cosmetic properties.

Thus, there is a real need to provide compositions, such as compositions for washing and/or conditioning keratin fibres, and in particular human keratin fibres, that make it possible to overcome the drawbacks described above, i.e. which effectively remove dirt and excess sebum and enhance cosmetic properties of said fibres, such as softness, smoothness, manageability and disentangling. These cosmetic properties may also be long-lasting.

In particular, the composition should give a satisfactory silicone deposition onto the keratin fibres and should improve hair disentangling.

These objectives are achieved with the present invention, a subject-matter of which is a composition comprising:
a) an oil-in-water emulsion having D50 particle size of less than 350 nm and comprising:
   - a silicone mixture comprising (i) a trialkylsilyl terminated dialkylpolysiloxane having a viscosity of from 40,000 to less than 100,000 mPa.s at 25°C and (ii) an amino silicone having a viscosity of from 1,000 to 15,000 mPa.s at 25°C and an amine value of from 2 to 10 mg of KOH per gram of amino silicone,
   - a mixture of emulsifiers comprising one or more nonionic emulsifiers, wherein the mixture of emulsifiers has a HLB value of from 10 to 16, and
   - water;
b) one or more polymers comprising one or more cationic or quaternized acrylamide and/or methacrylamide unit(s);
c) one or more cationic polysaccharides, different from polymers b);
d) optionally one or more anionic surfactants; and
e) optionally one or more amphoteric or zwitterionic surfactants;
wherein the weight ratio of the total amount of said cationic polysaccharide(s) c) to the total amount of said polymer(s) b) comprising one or more cationic or quaternized acrylamide and/or methacrylamide unit(s) ranges from 4 to 20.

This composition, when applied onto keratin fibres, in particular human keratin fibres such as hair and more particularly the sensitized hair, leads to an improvement of the condition and quality of hair, in terms of hair feel (e.g. smooth feel, soft feel, conditioned feel) and hair manageability (e.g., no or less frizz, styleability/shapeability, combing, disentangling, desirable volume).

In addition, the composition according to the invention makes it possible to significantly improve the silicone deposition onto the keratin fibres.

Even more surprisingly, it has been discovered that the silicone deposition onto the hair is significantly higher with the composition according to the invention compared with a similar composition which does not comprise said cationic polymers association.

The composition according to the invention has a good detergent power.

It has also been noted that hair treated with the composition according to the invention is particularly clean and light (no build-up).

The observed properties of the composition according to the invention are particularly long-lasting.

The present invention also relates to a process for treating, in particular for washing and/or conditioning, keratin fibres, preferably human keratin fibres such as the hair, comprising the application onto keratin fibres of this composition.

Another subject-matter of the invention is the use of the composition according to the invention for washing and/or conditioning keratin fibres, preferably the hair.

Other subjects, characteristics, aspects and advantages of the invention will emerge even more clearly on reading the description and the example that follows.

In the present description, and unless otherwise indicated:
- the expression "at least one" is equivalent to the expression "one or more" and can be replaced therewith;
- the expression "between" is equivalent to the expression "ranging from" and can be replaced therewith, and implies that the limits are included;
- according to the present application, the term "keratin fibres" preferably denotes human keratin fibres, and more preferentially the hair;

### The oil-in-water emulsion

The composition according to the present invention comprises an oil-in-water (or silicone-in-water) emulsion having D50 particle size of less than 350 nm and containing:
- a silicone mixture comprising (i) a trialkylsilyl terminated dialkylpolysiloxane having a viscosity of from 40,000 to less than 100,000 mPa.s at 25°C and (ii) an amino silicone having a viscosity of from 1,000 to 15,000 mPa.s at 25°C and an amine value of from 2 to 10 mg of KOH per gram of amino silicone,
- a mixture of emulsifiers comprising one or more nonionic emulsifiers, wherein the mixture of emulsifiers has a HLB value of from 10 to 16, and
- water.

In the oil-in-water emulsion, or silicone-in-water emulsion, one liquid phase (the dispersed phase) is dispersed in the other liquid phase (the continuous phase); in the present invention, the silicone mixture, or silicone phase, is dispersed in the continuous aqueous phase.

### - (i) trialkylsilyl terminated dialkylpolysiloxanes

The silicone mixture comprises a trialkylsilyl terminated dialkylpolysiloxane that is preferably of formula (IX):

R'₃SiO(R'₂SiO)ₚSiR'₃

wherein:
- R', same or different, is a monovalent hydrocarbon radical having from 1 to 18 carbon atoms, preferably from 1 to 6 carbon atoms, even better from 1 to 3 carbon atoms, more preferably methyl, and
- p is an integer of from 500 to 2,000, preferably of from 1,000 to 2,000.;

The trialkylsilyl terminated (or end-blocked or α,ω-position) dialkylpolysiloxanes according to the invention have a viscosity of from 40,000 to less than 100,000 mPa.s (100,000 excluded) at 25°C, preferably a viscosity of from 40,000 to 70,000 mPa.s at 25°C, more preferably a viscosity of from 51,000 to 70,000 mPa.s at 25°C.

The trialkylsilyl terminated dialkylpolysiloxanes according to the invention are preferably linear but may contain additionally to the R'₂SiO_{2/2} units (D-units) in formula (IX), RSiO_{3/2} units (T-units) and/or SiO_{4/2} units (Q-units), wherein R', same or different, is a monovalent hydrocarbon radical having from 1 to 18 carbon atoms.

Preferably, R', same or different, are alkyl radicals, preferably C₁-C₁₈ alkyl radicals, such as the methyl, ethyl, n-propyl, isopropyl, 1-n-butyl, 2-n-butyl, isobutyl, tert-butyl, n-pentyl, isopentyl, neopentyl and tert-pentyl radicals, hexyl radicals, such as the n-hexyl radical, heptyl radicals such as the n-heptyl radical, octyl radicals such as the n-octyl radical and isooctyl radicals, such as the 2,2,4-trimethylpentyl radical, nonyl radicals, such as the n-nonyl radicals, decyl radicals, such as the n-decyl radical, dodecyl radicals, such as the n-dodecyl radical, and octadecyl radicals, such as the n-octadecyl radical; alkenyl radicals such as the vinyl and ally radical; cycloalkyl radicals, such as the cyclopentyl, cyclohexyl, cycloheptyl and methylcyclohexyl radicals; aryl radicals, such as the phenyl, naphthyl, anthryl and phenanthryl radical; alkaryl radicals, such as the o-, m- and p-tolyl radicals, xylyl radicals and ethylphenyl radicals; and aralkyl radicals such as the benzyl radical and the a- and the b-phenylethyl radical. Most preferred is the methyl radical.

Preferably, the trialkylsilyl terminated dialkylpolysiloxanes are trimethylsilyl terminated PDMS (polydimethylsiloxanes or dimethicones).

### (ii) amino silicones

The silicone mixture comprises an amino silicone that are preferably of formula (X):

XR₂Si(OSiAR)ₙ(OSiR₂)ₘOSiR₂X

wherein:
- R, same or different, is a monovalent hydrocarbon radical having from 1 to 18 carbon atoms, preferably from 1 to 6 carbon atoms, even better from 1 to 3 carbon atoms, more preferably methyl;
- X, same or different, is R or a hydroxyl (OH) or a C₁-C₆-alkoxy group; preferably X is R, i.e. a monovalent hydrocarbon radical having from 1 to 18 carbon atoms, preferably from 1 to 6 carbon atoms, even better from 1 to 3 carbon atoms, more preferably methyl;
- A is an amino radical of the formula -R¹-[NR²-R³-]ₓNR²₂, or the protonated amino forms of said amino radical, wherein R¹ is a C₁-C₆-alkylene radical, preferably a radical of the formula -CH₂CH₂CH₂- or -CH₂CH(CH₃)CH₂-, R², same or different, is a hydrogen atom or a C₁-C₄-alkyl radical, preferably a hydrogen atom, R³ is a C₁-C₆-alkylene radical, preferably a radical of the formula -CH₂CH₂-, and x is 0 or 1; and
- m+n is an integer from 50 to about 1000, preferably from 50 to 600.

Preferably, A is an amino radical of the formula -R¹-[NR²-R³-]ₓNR²₂, or the protonated amino forms of said amino radical, wherein R¹ is -CH₂CH₂CH₂- or -CH₂CH(CH₃)CH₂-, R² are hydrogen atoms, R³ is -CH₂CH₂-, and x is 1.

Preferably, R, same or different, are alkyl radicals, preferably C₁-C₁₈ alkyl radicals, such as the methyl, ethyl, n-propyl, isopropyl, 1-n-butyl, 2-n-butyl, isobutyl, tert-butyl, n-pentyl, isopentyl, neopentyl and tert-pentyl radicals, hexyl radicals, such as the n-hexyl radical, heptyl radicals such as the n-heptyl radical, octyl radicals such as the n-octyl radical and isooctyl radicals, such as the 2,2,4-trimethylpentyl radical, nonyl radicals, such as the n-nonyl radicals, decyl radicals, such as the n-decyl radical, dodecyl radicals, such as the n-dodecyl radical, and octadecyl radicals, such as the n-octadecyl radical; alkenyl radicals such as the vinyl and ally radical; cycloalkyl radicals, such as the cyclopentyl, cyclohexyl, cycloheptyl and methylcyclohexyl radicals; aryl radicals, such as the phenyl, naphthyl, anthryl and phenanthryl radical; alkaryl radicals, such as the o-, m- and p-tolyl radicals, xylyl radicals and ethylphenyl radicals; and aralkyl radicals such as the benzyl radical and the a- and the b-phenylethyl radical. Most preferred is the methyl radical.

The amino silicones according to the invention have a viscosity of from 1,000 to 15,000 mPa.s at 25°C, preferably of from 1,500 to 15,000 mPa.s.

The amino silicones according to the invention have an amine value of from 2 to 10 mg of KOH per gram of amino silicone, preferably of from 3.5 to 8 mg.

The mole percent of amine functionality is preferably in the range of from about 0.3 to about 8%.

Examples of amino silicones useful in the silicone mixture according to the invention include trialkylsilyl terminated amino silicone.

Most preferably, amino silicones are trimethylsilyl terminated aminoethylaminopropylmethylsiloxane, most preferably trimethylsilyl terminated aminoethylaminopropylmethylsiloxane - dimethylsiloxane copolymers. The amino radical A can be protonated partially or fully by adding acids to the amino silicone, wherein the salt forms of the amino radical are obtained. Examples of acids are carboxylic acids with 3 to 18 carbon atoms which can be linear or branched, such as formic acid, acetic acid, propionic acid, butyric acid, pivalic acid, sorbic acid, benzoic acid, salicylic acid. The acids are preferably used in amounts of from 0.1 to 2.0 mol per 1 mol of amino radical A in the amino silicone of formula (X).

The silicone mixture preferably comprises (i) one or more trialkylsilyl terminated dialkylpolysiloxanes having a viscosity of from 40,000 to less than 100,000 mPa.s at 25°C in a quantity of from 70 to 90% by weight, preferably from 75 to 85% by weight and (ii) one or more amino silicones having a viscosity of from 1,000 to 15,000 mPa.s at 25°C and an amine value of from 2 to 10 mg of KOH per gram of amino silicone, in a quantity of from 10 to 30% by weight, preferably from 15 to 25% by weight, relative to the total weight of the silicone mixture.

### - mixture of emulsifiers

The oil-in-water emulsion further comprises a mixture of emulsifiers that comprises one or more nonionic emulsifiers. It could optionally comprise one or more cationic surfactants.

The mixture of emulsifiers has a HLB value from 10 to 16.

The nonionic emulsifiers can be chosen among the nonionic surfactants as described hereunder.

Mention may be made of alcohols, α-diols and (C₁₋₂₀)alkylphenols, these compounds being polyethoxylated and/or polypropoxylated and/or polyglycerolated, the number of ethylene oxide and/or propylene oxide groups possibly ranging from 1 to 100, and the number of glycerol groups possibly ranging from 2 to 30; or alternatively these compounds comprising at least one fatty chain comprising from 8 to 30 carbon atoms and especially from 16 to 30 carbon atoms.

Mention may also be made of condensates of ethylene oxide and of propylene oxide with fatty alcohols; polyethoxylated fatty amides preferably having from 2 to 30 ethylene oxide units, polyglycerolated fatty amides comprising on average from 1 to 5, and in particular from 1.5 to 4, glycerol groups; ethoxylated fatty acid esters of sorbitan preferably containing from 2 to 40 ethylene oxide units, fatty acid esters of sucrose, polyoxyalkylenated and preferably polyoxyethylenated fatty acid esters containing from 2 to 150 mol of ethylene oxide, including oxyethylenated plant oils, N-(C₆₋₂₄ alkyl)glucamine derivatives, amine oxides such as (C₁₀₋₁₄ alkyl)amine oxides or N-(C₁₀₋₁₄ acyl)aminopropylmorpholine oxides.

Mention may also be made of nonionic surfactants of alkyl(poly)glycoside type, represented especially by the following general formula:

R₁O-(R₂O)ₜ-(G)ᵥ

in which:
- R₁ represents a linear or branched alkyl or alkenyl radical comprising 6 to 24 carbon atoms and especially 8 to 18 carbon atoms, or an alkylphenyl radical whose linear or branched alkyl radical comprises 6 to 24 carbon atoms and especially 8 to 18 carbon atoms;
- R₂ represents an alkylene radical comprising 2 to 4 carbon atoms,
- G represents a sugar unit comprising 5 to 6 carbon atoms,
- t denotes a value ranging from 0 to 10 and preferably 0 to 4,
- v denotes a value ranging from 1 to 15 and preferably 1 to 4.

Preferably, the alkylpolyglycoside surfactants are compounds of the formula described above in which:
- R₁ denotes a linear or branched, saturated or unsaturated alkyl radical comprising from 8 to 18 carbon atoms,
- R₂ represents an alkylene radical comprising 2 to 4 carbon atoms,
- t denotes a value ranging from 0 to 3 and preferably equal to 0,
- G denotes glucose, fructose or galactose, preferably glucose;
- the degree of polymerization, i.e. the value of v, possibly ranging from 1 to 15 and preferably from 1 to 4; the mean degree of polymerization more particularly being between 1 and 2.

The glucoside bonds between the sugar units are generally of 1-6 or 1-4 type and preferably of 1-4 type. Preferably, the alkyl(poly)glycoside surfactant is an alkyl(poly)glucoside surfactant. C₈/C₁₆ alkyl(poly)glycosides 1,4, and especially decyl glucosides and caprylyl/capryl glucosides, are most particularly preferred.

Among the commercial products, mention may be made of the products sold by the company COGNIS under the names PLANTAREN^{®} (600 CS/U, 1200 and 2000) or PLANTACARE^{®} (818, 1200 and 2000); the products sold by the company SEPPIC under the names ORAMIX CG 110 and ORAMIX NS 10; the products sold by the company BASF under the name LUTENSOL GD 70, or else the products sold by the company CHEM Y under the name AG10 LK.

The nonionic emulsifiers could preferably be chosen among ethoxylated aliphatic alcohols, polyoxyethylene surfactants, carboxylic esters, polyethylene glycol esters, sorbitol ester and their ethoxylated derivatives, glycol esters of fatty acids, carboxylic amides, monoalkanolamine condensates, polyoxyethylene fatty acid amides.

Preferably, nonionic emulsifiers are selected from:
(i) polyoxyalkylene alkyl ethers, especially (poly)ethoxylated fatty alcohols of formula:

   R₃-(OCH₂CH₂)_{c}OH

   with:
   - R₃ representing a linear or branched C₈-C₄₀ alkyl or alkenyl group, preferably C₈-C₃₀ alkyl or alkenyl group, optionally substituted with one or more hydroxyl groups, and
   - c being an integer between 1 and 200 inclusive, preferentially between 2 and 150 and more particularly between 4 and 50, most preferably between 8 and 20.
      The (poly)ethoxylated fatty alcohols are more particularly fatty alcohols comprising from 8 to 22 carbon atoms, oxyethylenated with 1 to 30 mol of ethylene oxide (1 to 30 OE);
(ii) polyoxyalkylene (C₈-C₃₂)alkylphenyl ethers,
(iii) polyoxyalkylene sorbitan (C₈-C₃₂) fatty acid esters, especially polyethoxylated fatty acid esters of sorbitan preferably containing from 2 to 40 ethylene oxide units, most preferably from 2 to 20 ethylene oxide units; preferably polyoxyethylenated sorbitan (C₁₀-C₂₄) fatty acid esters preferably containing from 2 to 40 ethylene oxide units, most preferably from 2 to 20 ethylene oxide units; and
(iv) polyoxyethylenated (C₈-C₃₂) fatty acid esters containing for example from 2 to 150 mol of ethylene oxide; preferably polyoxyethylenated (C₁₀-C₂₄) fatty acid esters containing for example from 2 to 150 mol of ethylene oxide.

Preferably, the nonionic emulsifiers could be selected from alkyl ether of polyalkyleneglycol and alkyl esters of polyalkyleneglycol; preferably of polyethyleneglycol (PEG).

Some useful emulsifiers are:
- polyethyleneglycol octyl ether; polyethyleneglycol lauryl ether; polyethyleneglycol tridecyl ether; polyethyleneglycol cetyl ether; polyethyleneglycol stearyl ether; among these, mention may be made more particularly of trideceth-3, trideceth-10 and steareth-6.
- polyethyleneglycol nonylphenyl ether; polyethyleneglycol dodecylphenyl ether; polyethyleneglycol cetylphenyl ether; polyethyleneglycol stearylphenyl ether;
- polyethyleneglycol sorbitan monostearate, polyethyleneglycol sorbitan monooleate.
- polyethyleneglycol stearate, and especially PEG-100 stearate.

Most preferably, the nonionic emulsifiers are chosen among steareth-6, PEG-100 stearate, trideceth-3 and trideceth-10 and their mixture; preferably, all these emulsifiers are present in the mixture of emulsifiers.

The mixture of emulsifiers could comprise one or more cationic emulsifiers that could be selected among tetraalkylammonium halides, tetraarylammonium halides, tetraalkylarylammonium halides, and their salts; quaternary ammonium compounds including salts; preferably, the cationic emulsifiers could be chosen among cetrimonium halides or behentrimonium halides, such as chloride.

The oil-in-water emulsion preferably comprises the mixture of emulsifiers in a total amount of from 5 to 15% by weight, more preferably of from 8 to 15% by weight, most preferably of from 10 to 12% by weight, relative to the total weight of the emulsion.

The oil-in-water emulsion preferably comprises nonionic emulsifiers in a total amount of from 5 to 15% by weight, more preferably of from 8 to 15% by weight, most preferably of from 10 to 12% by weight, relative to the total weight of the emulsion.

The oil-in-water emulsion preferably comprises cationic emulsifiers, when present, in a total amount of from 0.5 to 1.5% by weight, relative to the total weight of the emulsion.

The oil-in-water emulsion preferably comprises the silicone mixture in a total amount of from 40 to 60% by weight, more preferably of from 45 to 55% by weight, relative to the total weight of the emulsion.

The oil-in-water emulsion preferably comprises the trialkylsilyl terminated dialkylpolysiloxane(s) in a total amount of from 35 to 45% by weight, more preferably of from 38-42% by weight, relative to the total weight of the emulsion.

The oil-in-water emulsion preferably comprises the amino silicone(s) in a total amount of from 5 to 15% by weight, more preferably of from 8-12% by weight, relative to the total weight of the emulsion.

The oil-in-water emulsion comprises water preferably in an amount of from 25 to 50% by weight, more preferably of from 30 to 45% by weight, most preferably of from 35 to 42% by weight, relative to the total weight of the emulsion.

The oil-in-water emulsion could additionally comprise a biocide, such as phenoxyethanol, that could be present in the emulsion in a quantity of from 0.5 to 1% by weight, relative to the total weight of the emulsion.

A method of preparation of the oil-in-water emulsion preferably comprises:
- a step of mixing one or more trialkylsilyl terminated dialkylpolysiloxanes of viscosity of from 40,000 to less than 100,000 mPa.s at 25°C and one or more amino silicones of viscosity of from 1,000 to 15,000 mPa.s at 25°C and an amine value of from 2 to 10 mg of KOH per gram of amino silicone, at a temperature of from 15°C to 40°C, preferably at 25°C, to obtain a mixed silicone fluid, then
- a step of adding a mixture of emulsifiers comprising one or more nonionic emulsifiers, wherein the mixture of emulsifiers has a HLB value from 10 to 16, to the mixed silicone fluid to obtain a silicone-emulsifier-mixture, then
- a step of homogenizing the silicone-emulsifier-mixture followed by
- a step of adding, preferably step-wise, water, preferably demineralized water, to obtain an oil-in-water emulsion having D50 particle size of less than 350 nm.

The method of preparation of the oil-in-water emulsion could further comprise an additional step of adding a biocide. Biocide could be added for preserving the emulsion against microbial contamination. The biocide could be added at the level of for preserving emulsion against microbial contamination and obtaining the said emulsion. The quantity of the biocide depends on the type of biocide and as recommended by the manufacturer.

The preparation of the mixture of emulsifiers could be made by mixing one or more nonionic emulsifiers.

The pH of the oil-in-water emulsion after neutralization (i.e. after addition of the biocide) is preferably of from 4 to 6.

The oil-in-water emulsion has D50 particle size of less than 350 nm, preferably from 100 to 300 nm, more preferably from 150 to 250 nm, even more preferentially from 150 to 225 nm, and most preferably from 160 to 200 nm. It corresponds to the average hydrodynamic particle diameter. The D50 particle size is expressed in volume. The D50 particle size could be measured by using a device ZetaSizer from Malvern, UK, model Nano-ZS, which is based on the Photon Correlation Spectroscopy (PCS) method.

### - Particle size measurement

Emulsion particle size could be measured by using a device ZetaSizer from Malvern, UK, model Nano-ZS which is based on the Photon Correlation Spectroscopy (PCS) method. The D50 value of particle size (average hydrodynamic particle diameter) is measured, wherein the evaluating algorithm is "cumulants analysis".

For example, take 0.5 g of the emulsion sample in a 250 ml beaker, 100 ml of demineralized water is poured into it and then mixed properly to get the sample test solution. The sample test solution is poured in the cuvette cell and is put into the slot of the instrument to measure the particle size of the emulsion. D50 is defined as the value of the particle diameter at 50% in the cumulative distribution. For example, if D50=170 nm, then 50% of the particles in the sample are larger than 170 nm, and 50% smaller than 170 nm or about 50% by volume of all droplets in said emulsion is 170 nm.

### - Viscosity measurement

The viscosity, especially of the silicones or of the emulsion, is measured at 25°C and at atmospheric pressure.

For viscosities between 1000 to 40,000 mPa.s at 25°C: the viscosity could be measured with an Anton Paar Rheometer; model MCR101, geometry single gap cylinder: CC27 spindle and shear rate of 1 s⁻¹ for 2 minutes, at 25°C.

For viscosities between 40,000 to 100,000 mPa.s at 25°C: the viscosity could be measured with an Anton Paar Rheometer; model MCR101, 25-6 cone (Cone-plate geometry: 25 mm dia. / 6° cone); the "Zero gap" setting being made and with a shear rate of 1 s⁻¹ for 2 minutes, at 25°C.

Three measurements are made for each sample and the viscosity value is taken at 60 seconds. MCR Rheometer Series products work as per USP (US Pharmacopeia Convention) 912 - Rotational Rheometer methods.

### - Amine value measurement

The amine value is determined by acid-base titration using a potentiometer [Make: Veego; Model: VPT-MG]. 0.6 g of sample is taken in a 500 ml beaker and a toluene-butanol 1:1 mixture is added and stirred to mix the sample thoroughly; then the sample solution is titrated with a 0.1(N) HCl solution. A determination of the blank value with the toluene-butanol 1:1 mixture is also done. The calculation of the amine value is done by the above-mentioned potentiometer.

The amine value is calculated according to the formula: $56.11 \times \left(V-{V}_{Blank}\right) \times N / W mg KOH / g of sample ,$ Wherein
V= Volume of HCl required in ml, V_{Blank}= Volume of HCl for blank value (without sample) with the toluene-butanol 1:1 mixture in ml; N= Normality of HCl, i.e. 0.1 N, W= weight of the sample taken in gram.

### - HLB Value

The term HLB is well known to those skilled in the art, and denotes the hydrophilic-lipophilic balance of a surfactant or emulsifier. In the present invention, HLB values refer to the values at 25°C and at atmospheric pressure.

The HLB can be measured by experimental determination or can be calculated.

Calculation of HLB value of nonionic surfactant is calculated according to the equation: HLB = (E + P)/5, with E being the weight percentage of oxyethylene content and P being the weight percentage of polyhydric alcohol content, described in to the publication Griffin, J. Soc. Cosm. Chem. 1954 (vol.5, n°4), pp.249-256.

It can also experimentally be determined according to the book of F. Puisieux and M. Seiller, entitled "Galenica 5: Les systèmes disperses - Tome I - Agents de surface et émulsions - Chapitre IV - Notions de HLB et de HLB critique, pp.153-194 - paragraph 1.1.2. Determination de HLB par voie experimentale [Experimental determination of HLB], pp.164-180".

The calculated HLB is the preferred HLB values that should be taken into account.

Said calculated HLB could be defined as being the following: 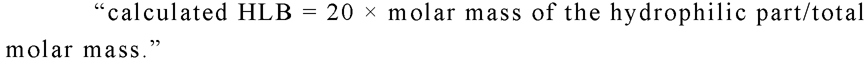 "calculated HLB = 20 × molar mass of the hydrophilic part/total molar mass."

For an oxyethylenated fatty alcohol, the hydrophilic part corresponds to the oxyethylene units condensed onto the fatty alcohol and the "calculated HLB" then corresponds to the "Griffin HLB" as defined hereabove.

For an ester or an amide, the hydrophilic part is naturally defined as being beyond the carbonyl group, starting from the fatty chain(s).

For ionic surfactants/emulsifiers, the HLB value of individual surfactant/emulsifier can be calculated applying the Davies formula as described in Davies JT (1957), "A quantitative kinetic theory of emulsion type, I. Physical chemistry of the emulsifying agent", Gas/Liquid and Liquid/Liquid Interface (Proceedings of the International Congress of Surface Activity): 426-438.

According to the formula, the HLB is derived by summing the hydrophilic/hydrophobic contribution afforded by the structural components of the emulsifier: 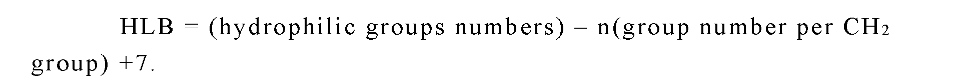 HLB = (hydrophilic groups numbers) - n(group number per CH2 group) +7.

Approximate HLB values for some cationic emulsifiers are given in Table IV, in "Cationic emulsifiers in cosmetics", GODFREY, J. Soc. Cosmetic Chemists (1966) 17, pp17-27.

When two emulsifiers A and B of known HLB are blended for use, the HLB_{Mix} is said to be the required HLB for the mixture. This is expressed by the equation (W_{A}HLB_{A} + W_{B}HLB_{B})/ (W_{A} + W_{B}) = HLB_{Mix}, where W_{A} = the amount (weight) of the first emulsifier (A) used, and W_{B} = the amount (weight) of the second emulsifier (B); HLB_{A}, HLB_{B} = the assigned HLB values for emulsifiers A and B; HLB_{Mix} = the HLB of the mixture.

Said oil-in-water emulsion is for example described in WO 2017/108824.

The composition according to the invention preferably comprises the oil-in-water emulsion a) in an amount ranging from 0.1% to 20% by weight, more preferably from 0.3 % to 15% by weight, even more preferably from 0.5% to 12% by weight, better from 1 to 10%, even better from 2 to 8% by weight, relative to the total weight of the composition.

The composition according to the invention preferably comprises the trialkylsilyl terminated dialkylpolysiloxane(s) having a viscosity of from 40,000 to less than 100,000 mPa.s at 25°C in a total amount ranging from 0.1% to 8% by weight, more preferably from 0.2 % to 5% by weight, even more preferably from 0.5% to 4% by weight, better from 1 to 3% by weight, relative to the total weight of the composition.

The composition according to the invention preferably comprises the amino silicone(s) having a viscosity of from 1,000 to 15,000 mPa.s at 25°C and an amine value of from 2 to 10 mg of KOH per gram of amino silicone, in a total amount ranging from 0.1% to 5% by weight, more preferably from 0.2 % to 3% by weight, even more preferably from 0.3% to 2% by weight, better from 0.4 to 1% by weight, relative to the total weight of the composition.

### The polymers comprising one or more cationic or quaternized acrylamide and/or methacrylamide unit(s)

The composition according to the invention comprises one or more polymers comprising one or more cationic or quaternized acrylamide and/or methacrylamide unit(s).

These polymers are non-silicones, i.e. they do not contain any silicon (Si) atom.

Polymers comprising one or more cationic or quaternized (meth)acrylamide unit(s) can be cationic and/ or amphoteric polymers.

By "cationic and/or amphoteric polymer(s)", it is understood one or more cationic polymers, one or more amphoteric polymers or the mixture of one or more cationic polymers, and of one or more amphoteric polymers.

The polymer(s) comprising one or more cationic or quaternized (meth)acrylamide units may be chosen from cationic polymers, amphoteric polymers and mixtures thereof. Most preferably, they are chosen from cationic polymers.

The cationic charge density of the polymers comprising one or more cationic or quaternized (meth)acrylamide units may preferably be lower than or equal to 6 meq/g, more preferentially lower than or equal to 5 meq/g, and better still lower than or equal to 4 meq/g. This cationic charge density advantageously ranges from 0.5 to 6 meq/g, better still from 1 to 5 meq/g, and even more preferably from 1.5 to 4 meq/g.

The term "cationic polymer" means any polymer comprising cationic groups and/or groups that can be ionized to cationic groups, and not comprising anionic groups and/or groups that can be ionized to anionic groups. Preferably, the cationic polymer is hydrophilic or amphiphilic. The preferred cationic polymers are chosen from those that contain units comprising primary, secondary, tertiary and/or quaternary amine groups that may either form part of the main polymer chain or may be borne by a side substituent directly connected thereto.

The polymers comprising one or more cationic or quaternized (meth)acrylamide units that can be used in the present invention are preferably chosen from homopolymers or copolymers comprising at least one of the units of the following formulae: 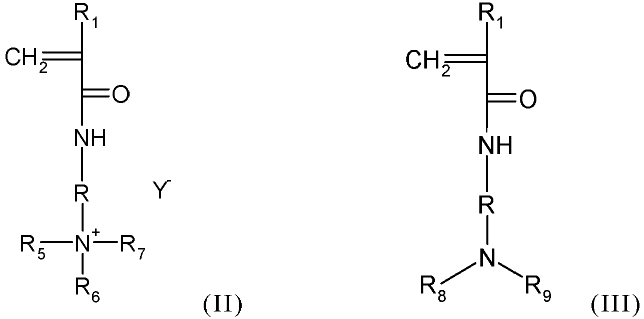 in which:
- R₁, which may be identical or different, denote a hydrogen atom or a CH₃ radical;
- R, which may be identical or different, denote a linear or branched C₁-C₁₂ alkyl radical, preferably a linear C₁-C₆ alkyl radical, optionally substituted by one or more hydroxyl radicals;
- R₅, R₆ and R₇, which may be identical or different, denote a linear or branched C₁-C₁₈ alkyl radical or a benzyl radical, preferably a linear or branched C₁-C₆ alkyl radical;
- R₈ and R₉, which may be identical or different, denote a hydrogen atom or a linear or branched C₁-C₆ alkyl radical, preferably methyl or ethyl; and
- Y⁻ denotes an anion derived from a mineral or organic acid or a halide, preferably bromide, chloride, acetate, borate, citrate, tartrate, bisulfate, bisulfite, methosulfate, sulfate or phosphate anion.

More particularly the polymers comprising cationic or quaternized (meth)acrylamide units are chosen from copolymers comprising at least one unit of formula (II) as defined previously, and more preferably comprising at least one unit of formula (II) in which R₁ denotes a hydrogen atom, R represents a linear alkyl group having 3 carbon atoms and R₅, R₆ and R₇ represent a methyl.

The cationic polymers comprising one or more cationic or quaternized (meth)acrylamide unit(s) may also contain one or more units derived from comonomers that may be selected from the families of acrylamides, methacrylamides, diacetone acrylamides, acrylic or methacrylic acids or esters thereof, vinyllactams such as vinylpyrrolidone or vinylcaprolactam, and vinyl esters, preferably selected from the families of acrylamide and methacrylamides, and more preferentially acrylamide or methacrylamide.

Among these homo- or co-polymers, mention may be made of:
- homopolymers of acrylamidopropyltrimonium halides, preferable Chloride, such as the product N-DURHANCE A-1000 from ASHLAND,
- copolymers of acrylamidopropyltrimonium halides, preferably chloride, and acrylamide, such as the product sold under the name Salcare^{®} SC 60 by the company BASF or sold under the name N-Hance SP 100 or N-Durhance AA2000 by the company Ashland, or the product sold under the name of N-Hance 4572 (e.g. Aqualon aqua 4572 conditioning polymer ) by the company Ashland, that is a mixture of guar hydroxypropyltrimonium chloride and of acrylamidepropyl-trimonium chloride/acrylamide copolymer,
- vinylpyrrolidone/methacrylamidopropyldimethylamine copolymers, such as those sold under the name STYLEZE CC 10 by ISP,
- quaternized vinylpyrrolidone/dimethylaminopropylmethacrylamide copolymers such as the product sold under the name GAFQUAT HS 100 by the company ISP.

The polymers comprising one or more cationic or quaternized (meth)acrylamide units that can be used in the present invention can also be chosen from amphoteric polymers.

The term "amphoteric polymer" means any polymer comprising cationic groups and/or groups that can be ionized to cationic groups, and comprising anionic groups and/or groups that can be ionized to anionic groups

Amphoteric polymers can be chosen more particularly from amphoteric polymers comprising a repetition of:
(i) one or more units derived from a monomer of (meth)acrylamide type,
(ii) one or more units derived from a monomer of (meth)acrylamidoalkyltrialkylammonium type, and
(iii) one or more units derived from an acidic monomer of (meth)acrylic acid type.

Preferably, the units derived from a monomer of (meth)acrylamide type (i) are units of structure (VI) below: 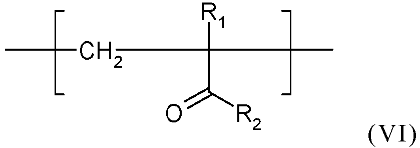 in which:
- R₁ denotes a hydrogen atom or CH₃ radical; and
- R₂ denotes a NR₃R₄ radical, wherein R₃ and R₄, which may be identical or different, denote a hydrogen atom or a linear or branched C₁-C₁₂ alkyl radical, optionally substituted by one or more hydroxyl radicals, preferably R₂ denotes an amino, a dimethylamino, a tert-butylamino, a dodecylamino or a -NH-CH₂OH radical.

Preferably, the said amphoteric polymer comprises a repetition of only one unit of formula (VI).

The unit derived from a monomer of (meth)acrylamide type of formula (VI) in which R₁ denotes a hydrogen atom and R₂ is an amino radical (NH₂) is particularly preferred. It corresponds to the acrylamide monomer per se.

Preferably, the units derived from a monomer of (meth)acrylamidoalkyltrialkylammonium type (ii) are units of structure (VII) below: 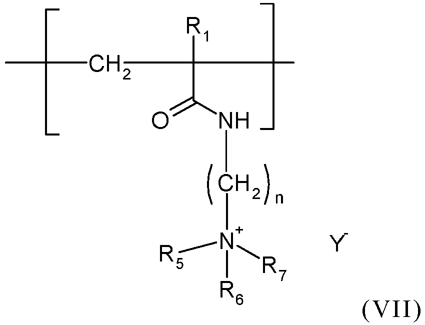 in which:
- R₁ denotes a hydrogen atom or CH₃ radical;
- R₅, R₆ and R₇, which may be identical or different, denote a linear or branched C₁-C₆ alkyl radical, preferably a linear or branched C₁-C₄ alkyl radical;
- n denotes an integer ranging from 1 to 6, preferably from 1 to 4; and
- Y⁻ denotes an anion derived from a mineral or organic acid or a halide, preferably bromide, chloride, acetate, borate, citrate, tartrate, bisulfate, bisulfite, methosulfate, sulfate or phosphate anion.

Preferably, the said amphoteric polymer comprises a repetition of only one unit of formula (VII).

Among these units derived from a monomer of (meth)acrylamidoalkyltrialkylammonium type of formula (VII), the ones that are preferred are those derived from the methacrylamidopropyltrimethylammonium chloride monomer, for which R₁ denotes a methyl radical, n is equal to 3, R₅, R₆ and R₇ denote a methyl radical, and Y⁻ denotes a chloride anion.

Preferably, the units derived from a monomer of (meth)acrylic acid type (iii) are units of formula (VIII): 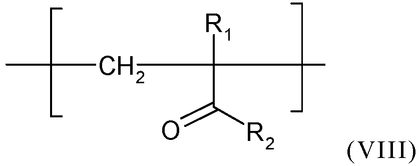 in which:
- R₁ denotes hydrogen atom or CH₃ radical; and
- R₂ denotes a hydroxyl radical or a NR₃R₄ radical, wherein R₃ and R₄, which may be identical or different, denote a hydrogen atom or a linear or branched C₁-C₁₂ alkyl radical optionally substituted by a sulfonic group (-SO₃H), preferably R₂ denotes a -NH-C(CH₃)₂-CH₂-SO₃H radical.

The preferred units of formula (VIII) correspond to the acrylic acid, methacrylic acid and 2-acrylamino-2-methylpropanesulfonic acid monomers.

Preferably, the unit derived from a monomer of (meth)acrylic acid type of formula (VIII) is that derived from acrylic acid, for which R₁ denotes a hydrogen atom and R₂ denotes a hydroxyl radical.

The acidic monomer(s) of (meth)acrylic acid type may be non-neutralized or partially or totally neutralized with an organic or mineral base.

Preferably, the said amphoteric polymer comprises a repetition of only one unit of formula (VIII).

According to a preferred embodiment of the invention, the amphoteric polymer(s) of this type comprise at least 30 mol% of units derived from a monomer of (meth)acrylamide type (i). Preferably, they comprise from 30 mol% to 70 mol% and more preferably from 40 mol% to 60 mol% of units derived from a monomer of (meth)acrylamide type.

The content of units derived from a monomer of (meth)acrylamidoalkyltrialkylammonium type (ii) may advantageously be from 10 mol% to 60 mol% and preferentially from 20 mol% to 55 mol%.

The content of units derived from an acidic monomer of (meth)acrylic acid type (iii) may advantageously be from 1 mol% to 20 mol% and preferentially from 5 mol% to 15 mol%.

According to a particularly preferred embodiment of the invention, the amphoteric polymer of this type comprises:
- from 30 mol% to 70 mol% and more preferably from 40 mol% to 60 mol% of units derived from a monomer of (meth)acrylamide type (i),
- from 10 mol% to 60 mol% and preferentially from 20 mol% to 55 mol% of units derived from a monomer of (meth)acrylamidoalkyltrialkylammonium type (ii), and
- from 1 mol% to 20 mol% and preferentially from 5 mol% to 15 mol% of units derived from a monomer of (meth)acrylic acid type (iii).

Amphoteric polymers of this type may also comprise additional units, other than the units derived from a monomer of (meth)acrylamide type, of (meth)acrylamidoalkyltrialkylammonium type and of (meth)acrylic acid type as described above.

However, according to a preferred embodiment of the invention, the said amphoteric polymers consist solely of units derived from monomers (i) of (meth)acrylamide type, (ii) of (meth)acrylamidoalkyltrialkylammonium type and (iii) of (meth)acrylic acid type.

As examples of amphoteric polymers that are particularly preferred, mention may be made of acrylamide/ methacrylamidopropyltrimethylammonium chloride/ acrylic acid terpolymers. Such polymers are listed in the CTFA Dictionary (International Cosmetic Ingredient Dictionary) under the name Polyquaternium 53. Corresponding products are especially sold under the names Merquat 2003 and Merquat 2003 PR by the company Nalco.

Another preferred type of amphoteric polymers is the polymer comprising a repetition of:
(i) one or more non ionic units derived from a monomer of (meth)acrylate type,
(ii) one or more units derived from a monomer of (meth)acrylamidoalkyltrialkylammonium type, and
(iii) one or more units derived from an acidic monomer of (meth)acrylic acid type.

The monomer of (meth)acrylamidoalkyltrialkylammonium type and the acidic monomer of (meth)acrylic acid type (monomers (ii) and (iii) respectively) are as described above.

The non ionic monomers (i) of (meth)acrylate type are preferably chosen from C₁-C₄ alky acrylates and methacrylates. A preferred monomer is methyl acrylate.

As particularly preferred examples of such amphoteric polymers, mention may be made of acrylic acid/ methylacrylamidopropyltrimethylammonium chloride/ methyl acrylates terpolymers. Such polymers are listed in the CTFA International Cosmetic Ingredient Dictionary under the name polyquaternium 47. Corresponding products are especially sold under the names Merquat 2001 and Merquat 2001N by the company Nalco.

The polymer(s) b) preferably comprises one or more cationic or quaternized (meth)acrylamide units and are more preferentially chosen from:
- (meth)acrylamido(C₁-C₆ alkyl)tri(C₁-C₄ alkyl) ammonium halide/ (meth)acrylamide copolymers, preferably (meth)acrylamidopropyltrimonium chloride/ (meth)acrylamide copolymers, and more preferably acrylamidopropyltrimonium chloride/ acrylamide copolymers,
- (meth)acrylamido(C₁-C₆ alkyl)tri(C₁-C₄ alkyl) ammonium halide /(meth)acrylamide/(meth)acrylic acid terpolymers, preferably (meth)acrylamidopropyltrimonium chloride / (meth)acrylamide / (meth)acrylic acid terpolymers, more preferably acrylamide/ methacrylamidopropyltrimethylammonium chloride/ acrylic acid terpolymers,
- (meth)acrylamido(C₁-C₆ alkyl)tri(C₁-C₄ alkyl) ammonium halide/ (C₁-C₆ alkyl) (meth)acrylate / (meth)acrylic acid terpolymers, preferably (meth)acrylamidopropyltrimonium chloride / (C₁-C₆ alkyl) (meth)acrylate / (meth)acrylic acid terpolymers; more preferably acrylic acid/ methylacrylamidopropyltrimethylammonium chloride/ methyl acrylates terpolymers,
- and mixtures thereof.

Even more preferentially, the polymer(s) comprising one or more cationic or quaternized (meth)acrylamide units are chosen from:
- (meth)acrylamido(C₁-C₆ alkyl)tri(C₁-C₄ alkyl) ammonium halide/ (meth)acrylamide copolymers, better (meth)acrylamide-propyltrimonium chloride/ (meth)acrylamide copolymers, and most preferably acrylamidopropyltrimonium chloride/ acrylamide copolymers.

Preferably, the total amount of polymer(s) b) comprising one or more cationic or quaternized (meth)acrylamide units present in the composition of the present invention advantageously ranges from 0.01 to 5% by weight, more preferentially from 0.015 to 4% by weight, even more preferentially from 0.02 to 2% by weight, better from 0.04 to 1% by weight, and even better from 0.05 to 0.5% by weight, relative to the total weight of the composition.

Preferentially, the composition could comprise the polymer(s) chosen from (meth)acrylamido(C₁-C₆ alkyl)tri(C₁-C₄ alkyl) ammonium halide/ (meth)acrylamide copolymers, in a total amount advantageously ranging from 0.01 to 5% by weight, more preferentially from 0.015 to 4% by weight, even more preferentially from 0.02 to 2% by weight, better from 0.04 to 1% by weight, and even better from 0.05 to 0.5% by weight, relative to the total weight of the composition.

### The cationic polysaccharides

The composition according to the invention comprises one or more cationic polysaccharides.

According to the invention, the cationic polysaccharide(s) c) used in the composition is(are) different from the polymers b) comprising one or more cationic or quaternized acrylamide and/or methacrylamide unit(s).

The term "cationic polysaccharide" means any polysaccharide comprising cationic groups and/or groups that can be ionized to cationic groups, and not comprising anionic groups and/or groups that can be ionized to anionic groups.

Among the cationic polysaccharides that can be used according to the invention, mention may be made more particularly of cellulose ether derivatives comprising quaternary ammonium groups, cationic cellulose copolymers or cellulose derivatives grafted with a water-soluble quaternary ammonium monomer and cationic galactomannan gums.

The cellulose ether derivatives comprising quaternary ammonium groups are especially described in French patent 1 492 597, and mention may be made of the polymers sold under the name UCARE POLYMER "JR" (JR 400 LT, JR 125 and JR 30M) or "LR" (LR 400 or LR 30M) by the company Dow chemical. These polymers are also defined in the CTFA dictionary as quaternary ammoniums of hydroxyethylcellulose that have reacted with an epoxide substituted with a trimethylammonium group. The polyquaternium-10 is, for example, one of these polymers.

Cationic cellulose copolymers or cellulose derivatives grafted with a water-soluble quaternary ammonium monomer are described especially in US patent 4 131 576, and mention may be made of hydroxyalkylcelluloses, for instance hydroxymethyl-, hydroxyethyl- or hydroxypropylcelluloses grafted, in particular, with a methacryloylethyltrimethylammonium, methacrylamidopropyltrimethylammonium or dimethyldiallylammonium salt. The commercial products corresponding to this definition are more particularly the products sold under the names CELQUAT L 200 and CELQUAT H 100 by the company National Starch.

The cationic galactomannan gums are described more particularly in US patents 3 589 578 and 4 031 307, and mention may be made of guar gums comprising cationic trialkylammonium groups, preferably C₁-C₆ trialkylammonium groups. Use is made, for example, of guar gums modified with a 2,3-epoxypropyltrimethylammonium salt (for example, chloride). Such products are especially sold under the names JAGUAR C13 S, JAGUAR C 15, JAGUAR C 17 or JAGUAR C162 by the company Rhodia.

Preferably, the cationic polysaccharide(s) is(are) chosen from cationic celluloses, cationic galactomannan gums, and mixtures thereof.

Preferentially, the cationic polysaccharide(s) is(are) chosen from cellulose ether derivatives comprising quaternary ammonium groups, guar gums comprising cationic trialkylammonium groups, and mixtures thereof.

More preferentially, the composition according to the invention comprises polyquaternium-10.

Preferably, the total amount of cationic polysaccharide(s) ranges from 0.01 to 5% by weight, more preferentially from 0.015 to 4% by weight, even more preferentially from 0.02 to 3% by weight, better from 0.05 to 2% by weight, and even better from 0.1 to 1% by weight, relative to the total weight of the composition.

Preferably, when the cationic polysaccharide(s) is(are) chosen from cellulose ether derivatives comprising quaternary ammonium groups, the total amount of cationic polysaccharide(s) from 0.01 to 5% by weight, more preferentially from 0.015 to 4% by weight, even more preferentially from 0.02 to 3% by weight, better from 0.05 to 2% by weight, and even better from 0.1 to 1% by weight, relative to the total weight of the composition.

Preferably, when the cationic polysaccharide is polyquaternium-10, the total amount of cationic polysaccharide ranges from 0.01 to 5% by weight, more preferentially from 0.015 to 4% by weight, even more preferentially from 0.02 to 3% by weight, better from 0.05 to 2% by weight, and even better from 0.1 to 1% by weight, relative to the total weight of the composition.

Preferably, when the cationic polysaccharide is a guar gum comprising cationic trialkylammonium groups, the total amount of cationic polysaccharide ranges from 0.01 to 5% by weight, more preferentially from 0.015 to 4% by weight, even more preferentially from 0.02 to 3% by weight, better from 0.05 to 2% by weight, and even better from 0.1 to 1% by weight, relative to the total weight of the composition.

The weight ratio of the total amount of said cationic polysaccharide(s) c) to the total amount of said polymer(s) b) comprising one or more cationic or quaternized acrylamide and/or methacrylamide unit(s) ranges from 4 to 20; and even better from 4 to 10.

Preferentially, the weight ratio of the total amount of cellulose ether derivatives comprising quaternary ammonium groups to the total amount of said polymers b) comprising one or more cationic or quaternized acrylamide and/or methacrylamide unit(s) ranges from to 20; and even better from 4 to 10.

More preferentially, the weight ratio of the total amount of polyquaternium-10 to the total amount of said polymers b) comprising one or more cationic or quaternized acrylamide and/or methacrylamide unit(s) ranges from 4 to 20; and even better from 4 to 10.

Even more preferentially, the weight ratio of the total amount of polyquaternium-10 to the total amount of (meth)acrylamido(C₁-C₆ alkyl)tri(C₁-C₄ alkyl) ammonium halide/ (meth)acrylamide copolymers ranges from 4 to 20; and even better from 4 to 10.

Preferentially, the weight ratio of the total amount of guar gum comprising cationic trialkylammonium groups to the total amount of said polymers b) comprising one or more cationic or quaternized acrylamide and/or methacrylamide unit(s) ranges from 4 to 20; and even better from 4 to 10.

### The anionic surfactants

Preferably, the composition according to the invention further comprises one or more anionic surfactants.

The term "anionic surfactant" means a surfactant comprising, as ionic or ionizable groups, only anionic groups.

In the present description, a species is termed as being "anionic" when it bears at least one permanent negative charge or when it can be ionized as a negatively charged species, under the conditions of use of the composition of the invention (for example the medium or the pH) and not comprising any cationic charge.

The anionic surfactants that may be used in the invention are different from the polymers b) as described previously.

The anionic surfactants may be sulfate, sulfonate and/or carboxylic (or carboxylate) surfactants. Needless to say, a mixture of these surfactants may be used.

It is understood in the present description that:
- carboxylate anionic surfactants comprise at least one carboxylic or carboxylate function (-COOH or -COO⁻) and may optionally also comprise one or more sulfate and/or sulfonate functions;
- the sulfonate anionic surfactants comprise at least one sulfonate function (-SO₃H or -SO₃⁻) and may optionally also comprise one or more sulfate functions, but do not comprise any carboxylate functions; and
- the sulfate anionic surfactants comprise at least one sulfate function but do not comprise any carboxylate or sulfonate functions.

The sulfate anionic surfactants that may be used comprise at least one sulfate function (-OSO₃H or -OSO₃⁻).

They may be chosen from the following compounds: alkyl sulfates, alkyl ether sulfates, alkylamido ether sulfates, alkylaryl polyether sulfates, monoglyceride sulfates; and also the salts of these compounds.

The alkyl groups of these compounds comprise from 6 to 30 carbon atoms, especially from 8 to 28, better still from 10 to 24 or even from 12 to 22 carbon atoms; the aryl group preferably denotes a phenyl or benzyl group.

These compounds are possibly polyoxyalkylenated, especially polyoxyethylenated, and then preferably comprising from 1 to 50 ethylene oxide units and better still from 2 to 10 ethylene oxide units.

When the anionic surfactant is in salt form, the said salt may be chosen from alkali metal salts, such as the sodium or potassium salt, ammonium salts, amine salts and in particular amino alcohol salts, and alkaline-earth metal salts, such as the magnesium salt.

Examples of amino alcohol salts that may be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)aminomethane salts.

Alkali metal or alkaline-earth metal salts and in particular the sodium or magnesium salts are preferably used.

More preferentially, the anionic surfactant(s) is(are) selected from the anionic surfactants of alkyl(ether) sulfate type, and better still from C₁₂-C₁₄ alkyl(ether) sulfate salts, and in particular lauryl ether sulfate salts.

The anionic surfactants suitable in the composition of the present invention can be oxyethylenated and then preferably comprise from 1 to 50 ethylene oxide units.

Preferably, the anionic surfactant(s) is(are) present in a total amount ranging from 0.1 % to 40% by weight, preferentially from 0.5% to 30%, more preferentially from 1% to 25% by weight, even more preferentially is greater than or equal to 5% by weight, better from 5 % to 20% by weight, even better from 10 to 15% by weight, relative to the total weight of the composition.

Preferably, when the one or more anionic surfactants are chosen from those of alkyl(ether) sulfate type, the one or more surfactants of alkyl(ether) sulfate type are present in a total amount ranging from 0.1 % to 40% by weight, more preferably from 0.5% to 30%, even more preferably from 1% to 25% by weight, even more preferentially is greater than or equal to 5% by weight, better from 5 % to 20% by weight, even better from 10 to 15% by weight, relative to the total weight of the composition.

Preferably, when the one or more anionic surfactants are chosen from C₁₂-C₁₄ alkyl(ether) sulfate salts, the one or more C₁₂-C₁₄ alkyl(ether) sulfate salts are present in a total amount ranging from 0.1 % to 40% by weight, more preferably from 0.5% to 30%, even more preferably from 1% to 25% by weight, even more preferentially is greater than or equal to 5% by weight, better from 5 % to 20% by weight, even better from 10 to 15% by weight, relative to the total weight of the composition.

Preferably, when the one or more anionic surfactants are chosen from lauryl ether sulfate salts, the one or more lauryl ether sulfate salts are present in a total amount ranging from 0.1 % to 40% by weight, more preferably from 0.5% to 30%, even more preferably from 1% to 25% by weight, even more preferentially is greater than or equal to 5% by weight, better from 5 % to 20% by weight, even better from 10 to 15% by weight, relative to the total weight of the composition.

### The amphoteric or zwitterionic surfactants

Preferably, the composition according to the invention further comprises one or more amphoteric or zwitterionic surfactants.

The amphoteric or zwitterionic surfactants are different from the polymers b) as described previously.

The amphoteric or zwitterionic surfactant(s) that may be used in the present invention may especially be secondary or tertiary aliphatic amine derivatives, optionally quaternized, in which the aliphatic group is a linear or branched chain containing from 8 to 22 carbon atoms, the said amine derivatives containing at least one anionic group, for instance a carboxylate, sulfonate, sulphate, phosphate or phosphonate group.

More preferentially, the amphoteric or zwitterionic surfactant(s) is(are) chosen from (C₈-C₂₀ alkyl)betaines such as cocoylbetaine, (C₈-C₂₀ alkyl)amido(C₂-C₈ alkyl)betaines such as cocoylamido-propylbetaine, and mixtures thereof.

Preferably, the one or more amphoteric or zwitterionic surfactants are present in a total amount ranging from 0.01 % to 25% by weight, more preferably from 0.1% to 20%, even more preferably from 0.5% to 15% by weight, better from 0.75% to 10% by weight, even better from 1 to 5% by weight, relative to the total weight of the composition.

According to a preferred embodiment of the invention, the composition according to the invention comprises one or more anionic surfactants and one or more amphoteric or zwitterionic surfactants, as described previously.

More preferably according to this embodiment, the composition comprises:
- one or more surfactants of alkyl(ether) sulfate type, in particular C₁₂-C₁₄ alkyl(ether) sulfate salts such as lauryl ether sulfate salts, and
- one or more amphoteric or zwitterionic surfactants chosen from (C₈-C₂₀ alkyl)betaines such as cocoylbetaine, (C₈-C₂₀ alkyl)amido(C₂-C₈ alkyl)betaines such as cocoylamido-propylbetaine, and mixtures thereof.

Preferably, the total amount of surfactants may range from 0.1% to 40% by weight, more preferably from 0.5 % to 30% by weight and even more preferably from 1 % to 25% by weight, better still from 5 to 20% by weight relative to the total weight of the composition.

Preferably, the composition according to the invention is a cosmetic composition, more preferably a hair composition such as a hair composition for cleansing and/or conditioning hair.

Optionally, the composition of the invention may also contain various additives conventionally used in hair compositions.

As additives that may be used in accordance with the invention, mention may be made of anionic or non-ionic polymers, antidandruff agents, anti-seborrhoea agents, agents for preventing hair loss and/or for promoting hair regrowth, fatty substances, vitamins and provitamins including panthenol, sunscreens, mineral or organic pigments, direct dyes, sequestrants, plasticizers, solubilizers, acidifying agents, mineral or organic thickeners, especially polymeric thickeners, opacifiers or nacreous agents, antioxidants, hydroxy acids, fragrances and preserving agents, and mixtures thereof.

Needless to say, a person skilled in the art will take care to select this or these optional additive(s) such that the advantageous properties intrinsically associated with the composition in accordance with the invention are not, or are not substantially, adversely affected by the envisaged addition(s).

The present invention also relates to a process for treating keratin fibres, preferably for washing and/or conditioning keratin fibres such as the hair, comprising a step of application onto the keratin fibres of a composition as described above.

Optionally, after the step of application, the composition according to the invention is removed after an optional leave-on time.

The leave-on time of the composition on the keratin fibres may range from a few seconds to 15 minutes, better still from 5 seconds to 10 minutes and even better still from 10 seconds to 5 minutes.

Preferably, the keratin fibres are not rinsed after the application onto the keratin fibres of the composition according to the invention, more preferentially in the 8 hours following the application.

The composition may be applied to wet or dry keratin fibres; preferably on wet keratin fibres.

The composition could be a shampoo, a conditioner or a hair mask.

Finally, the present invention relates to the use of a composition as described above for washing and/or conditioning keratin fibres, preferably the hair.

In the above description, all the preferred embodiments with regard to the components may be used individually or in combination.

The examples that follow serve to illustrate the invention.

### Examples:

In the examples that follow and unless otherwise indicated, the amounts are given as weight percentages of active material (AM) relative to the total weight of the composition.

### Example 1: Preparation of an oil-in-water emulsion

450 g of amino silicone fluid (trimethylsilyl-terminated aminoethyl-aminopropylmethylsiloxane - dimethylsiloxane copolymer with amine value of 7.2 mg of KOH/g sample, and a viscosity of 5,600 mPa.s at 25°C) were introduced in an emulsion tank. Stirring was started and 1,800 g of trimethylsilyl terminated dimethylsiloxane polymer fluid of viscosity 61,500 mPa.s at 25°C were introduced under stirring in the same tank. Both fluids were mixed for 2 hours at room temperature.

In a separate tank, 49 g of steareth-6 and 62 g of PEG-100 stearate were introduced and heated to 60°C. The temperature was maintained till both emulsifiers became liquid. Then 31 g of trideceth-3 and 350 g of trideceth-10 (80% of active material) were added. These nonionic emulsifiers mixture had an HLB value = 11.25.

Then 80 g water and 6.2 g glacial acetic acid were added to the tank and the mixing started. The mixing was continued till whole mass became a creamy paste. The whole paste was introduced in the emulsion tank. Homogenization was carried out for 30 minutes at room temperature. 79.6 g demineralized water were added and homogenization was carried out for 60 minutes. 72.7 g demineralized water were added and homogenization was carried out for 50 minutes. 197.4 g demineralized water were added and homogenization was carried out for 5 minutes. 294.3 g demineralized water were added and homogenization was carried out for 5 minutes. 180 g demineralized water were added and homogenization was carried out for 5 minutes. 180 g demineralized water were added and homogenization was carried out for 5 minutes. 197.4 g demineralized water were added and homogenization was carried out for 5 minutes. 197.4 g demineralized water were added and homogenize for 3 minutes. 228.5 g demineralized water were added and homogenization was carried out for 3 minutes. Lastly 40.5 g 2-phenoxyethanol were added as a biocide and homogenization was carried out for 3 minutes.

A stable oil-in-water emulsion having D50 particle size of 170 nm was obtained.

### Example 2:

The following composition A according to the invention was prepared from the ingredients indicated in Table 1 below (wt. % of active material).

**Table 1**

| **Ingredients** | **Composition A** (Invention) |
|---|---|
| Coco-betaine | 1.23 |
| Sodium laureth sulfate | 12.6 |
| Sodium chloride | 1.4 |
| Carbomer | 0.35 |
| Acrylamidopropyltrimonium chloride/acrylamide copolymer | 0.08 |
| Polyquaternium-10 | 0.4 |
| Dimethicone (and) Amodimethicone (and) Trideceth-10 (and) PEG-100 stearate (and) Steareth-6 (and) Trideceth-3 (of exemple 1) | 5 % of emulsion, i.e. 0.5% AM SiA + 2% AM of Si |
| Glycerin | 0.5 |
| Hexylene glycol | 0.6 |
| Glycol distearate | 0.13 |
| Mica | 0.04 |
| Preservatives, pH agent | qs |
| Water | Qs 100 |

The silicone deposition of composition A above has been evaluated on locks of natural hair and of medium bleached hair (ie. damaged hair).

The evaluation has been performed with the WDXRF Optim'x Thermofisher (Wavelength Dispersion) XRF system. The principle is based on radiation emissions characteristic of the chemical element, produced by the impact of high energy photons dispensed by an X-ray tube.

### Operational parameters:

- Gas Flow Helium - Argon/Methane (90/10)
- X-ray tube (Rh), Crystal PET and detector FPC
- Tension 25kv- 2mA
- 3 measurements / lock = 60s/ measurement
- Minimum sample size 250mg (2mm pieces)
*Protocol:* 0.4g of composition was applied for 1g of hair lock. The shampoo was messaged 6 times using fingers from root to tip, to generate foam. The lock was then rinsed under running water (25°C) 10 second. The lock was then dried at 45°C in an oven. This procedure was counted as 1 wash and then followed for 4 more washes to complete 5 wash cycle.

The silicone deposition of the composition A onto keratin fibres has been measured after one application and after 5 applications for each.

The results are shown in the table 2 below.

**Table 2**

| **Locks** | **Number of applications** | **Average Silicone deposition (ppm)** |
|---|---|---|
| Natural Hair | 1 | 2418 ± 21 |
| | 5 | 5579 ± 135 |
| Medium Bleached Hair | 1 | 1317 ± 36 |
| | 5 | 4118 ± 198 |

The composition A according to the invention shows substantial deposition of silicone on medium bleached hair as well as on natural hair.

### Example 3:

The following compositions B (invention) and C (comparative) was prepared from the ingredients indicated in Table 3 below (wt. % of active material).

**Table 3**

| **Ingredients** | **Composition B** (Invention) | **Composition C** (Comparative) |
|---|---|---|
| Coco-betaine | 1.5 | 1.5 |
| Sodium laureth sulfate | 13.9 | 13.9 |
| Sodium chloride | 1.4 | 1.4 |
| Carbomer | 0.2 | 0.2 |
| Acrylamidopropyltrimo nium chloride / acrylamide copolymer | 0.1 | 0.1 |
| Polyquaternium-10 | 0.5 | - |
| Dimethicone (and) Amodimethicone (and) Trideceth-10 (and) PEG-100 stearate (and) Steareth-6 (and) Trideceth-3 (of exemple 1) | 6% of emulsion, i.e. 0.6% AM SiA + 2.4% AM of Si | 6% of emulsion, i.e. 0.6% AM SiA + 2.4% AM of Si |
| Glycerin | 0.5 | 0.5 |
| Hexylene glycol | 0.15 | 0.15 |
| Cocos nucifera oil | 0.02 | 0.02 |
| Glycol distearate | 1.6 | 1.6 |
| Preservatives, pH agent | qs | qs |
| Water | Qs 100 | Qs 100 |

The hair locks were shampooed, rinsed with water and placed on a hot plate (30°C).

Then the composition to test was applied onto the hair lock and rinsed off under running water.

A wet hair lock (3g, 20 cm, medium bleached hair) to which 1.2g of composition B or C had been applied, was placed on a combing machine (Diastron MTT 175 by Dia-Stron Limited UK) and a comb with a sensor was put into the hair fibers.

Combing was performed by scanning the hair lock from root to tip, and measured friction force.

The measurements were performed 3 times per one wet hair lock. In total, the measurements were performed for 5 hair locks.

The maximum force was selected from the measured data of each hair lock. The average value of the 5 maximum forces was calculated in gram-force (gmf): 100 gmf corresponds to about 0.98 Newton.

The lower the force, the easier the keratin fibers are to comb / disentangle.
Conditions of the test: 20°C, 50° humidity
Combing speed = 1500 mm/minute

The results are shown in the tables 4 (dry combing) and 5 (wet combing) below.

**Table 4: Dry combing**

| **Compositions** | **Average value of the 5 maximum forces (in gmf)** |
|---|---|
| **B** (Invention) | 21.93 |
| **C** (Comparative) | 24.62 |

**Table 5: Wet combing**

| **Compositions** | **Average value of the 5 maximum forces (in gmf)** |
|---|---|
| **B** (Invention) | 65.27 |
| **C** (Comparative) | 76.19 |

It is observed that the composition B according to the invention provides a smoother feel, a better combing and a better disentangling of the hair than the comparative composition C.

## Claims

1. Composition comprising:
a) an oil-in-water emulsion having D50 particle size of less than 350 nm, wherein the particle size is measured by using a device ZetaSizer from Malvern, model Nano-ZS which is based on the Photon Correlation Spectroscopy (PCS) method, and comprising:
- a silicone mixture comprising (i) a trialkylsilyl terminated dialkylpolysiloxane having a viscosity of from 40,000 to less than 100,000 mPa.s at 25°C, the viscosity being measured at 25°C using an Anton Paar Rheometer; model MCR101, 25-6 cone ; the "Zero gap" setting being made and with a shear rate of 1 s¹ for 2 minutes, and
(ii) an amino silicone having a viscosity of from 1,000 to 15,000 mPa.s at 25°C, the viscosity being measured at 25°C using an Anton Paar Rheometer; model MCR101, geometry single gap cylinder: CC27 spindle and shear rate of 1 s¹ for 2 minutes, and an amine value of from 2 to 10 mg of KOH per gram of amino silicone,
- a mixture of emulsifiers comprising one or more nonionic emulsifiers, wherein the mixture of emulsifiers has a HLB value of from 10 to 16, and
- water;
b) one or more polymers comprising one or more cationic or quaternized acrylamide and/or methacrylamide unit(s); and
c) one or more cationic polysaccharides, different from polymers b);
wherein the weight ratio of the total amount of said cationic polysaccharide(s) c) to the total amount of said polymer(s) b) comprising one or more cationic or quaternized acrylamide and/or methacrylamide unit(s) ranges from 4 to 20.

2. Composition according to Claim 1, wherein the weight ratio of the total amount of said cationic polysaccharides c) to the total amount of said polymers b) comprising one or more cationic or quaternized acrylamide and/or methacrylamide unit(s) ranges from 4 to 10.

3. Composition according to any one of the preceding claims, wherein the trialkylsilyl terminated dialkylpolysiloxane (i) is of formula (IX):
R'₃SiO(R'₂SiO)ₚSiR'₃
wherein:
- R', same or different, is a monovalent hydrocarbon radical having from 1 to 18 carbon atoms, preferably from 1 to 6 carbon atoms, and
- p is an integer of from 500 to 2,000, preferably of from 1,000 to 2,000; and preferably is trimethylsilyl terminated PDMS.

4. Composition according to any one of the preceding claims, wherein the amino silicone (ii) is of formula (X):
XR₂Si(OSiAR)ₙ(OSiR₂)ₘOSiR₂X
wherein:
- R, same or different, is a monovalent hydrocarbon radical having from 1 to 18 carbon atoms, preferably from 1 to 6 carbon atoms,
- X, same or different, is R or a hydroxyl (OH) or a C₁-C₆-alkoxy group; preferably X is R,
- A is an amino radical of the formula -R¹-[NR²-R³-]ₓNR²₂, or the protonated amino forms of said amino radical, wherein R¹ is a C₁-C₆-alkylene radical, preferably a radical of the formula -CH₂CH₂CH₂- or - CH₂CH(CH₃)CH₂-, R², same or different, is a hydrogen atom or a C₁-C₄-alkyl radical, preferably a hydrogen atom, R³ is a C₁-C₆-alkylene radical, preferably a radical of the formula -CH₂CH₂-, and x is 0 or 1; and
- m+n is an integer from 50 to 1000, preferably from 50 to 600; preferably A is an amino radical of the formula -R¹-[NR²-R³-]ₓNR²₂, or the protonated amino forms of said amino radical, wherein R¹ is -CH₂CH₂CH₂- or -CH₂CH(CH₃)CH₂-, R² are hydrogen atoms, R³ is -CH₂CH₂-, and x is 1.

5. Composition according to any one of the preceding claims, wherein the mixture of emulsifiers comprises one or more nonionic emulsifiers chosen from:
(i) polyoxyalkylene alkyl ethers, especially (poly)ethoxylated fatty alcohols of formula: R₃-(OCH₂CH₂)_{c}OH with:
- R₃ representing a linear or branched C₈-C₄₀ alkyl or alkenyl group, preferably C₈-C₃₀ alkyl or alkenyl group, optionally substituted with one or more hydroxyl groups, and
- c being an integer between 1 and 200 inclusive, preferentially between 2 and 150;
and more particularly fatty alcohols comprising from 8 to 22 carbon atoms, oxyethylenated with 1 to 30 mol of ethylene oxide (1 to 30 OE);
(ii) polyoxyalkylene (C₈-C₃₂)alkylphenyl ethers;
(iii) polyoxyalkylene sorbitan (C₈-C₃₂) fatty acid esters, especially polyethoxylated fatty acid esters of sorbitan preferably containing from 2 to 40 ethylene oxide units, most preferably from 2 to 20 ethylene oxide units; preferably polyoxyethylenated sorbitan (C₁₀-C₂₄) fatty acid esters preferably containing from 2 to 40 ethylene oxide units, most preferably from 2 to 20 ethylene oxide units; and;
(iv) polyoxyethylenated (C₈-C₃₂) fatty acid esters containing for example from 2 to 150 mol of ethylene oxide; preferably polyoxyethylenated (C₁₀-C₂₄) fatty acid esters containing for example from 2 to 150 mol of ethylene oxide.

6. Composition according to any one of the preceding claims, wherein the oil-in-water emulsion a) has D50 particle size from 100 to 300 nm, preferably from 150 to 250 nm, more preferably from 150 to 225 nm, and even more preferentially from 160 to 200 nm, expressed in volume.

7. Composition according to any one of the preceding claims, wherein the polymer(s) comprising one or more cationic or quaternized acrylamide and/or methacrylamide unit(s) is(are) chosen from homopolymers or copolymers comprising at least one of the units of the following formulae: 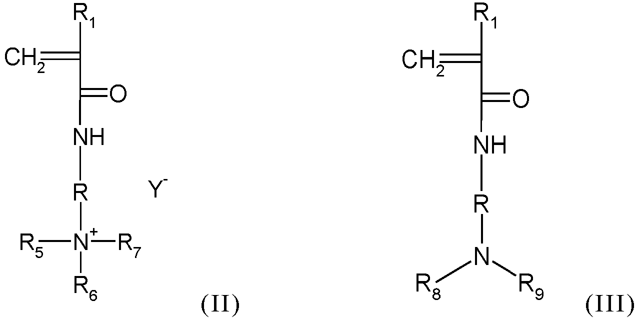 in which:
- R₁, which may be identical or different, denote a hydrogen atom or a CH₃ radical;
- R, which may be identical or different, denote a linear or branched C₁-C₁₂ alkyl radical, preferably a linear C₁-C₆ alkyl radical, optionally substituted by one or more hydroxyl radicals;
- R₅, R₆ and R₇, which may be identical or different, denote a linear or branched C₁-C₁₈ alkyl radical or a benzyl radical, preferably a linear or branched C₁-C₆ alkyl radical;
- R₈ and R₉, which may be identical or different, denote a hydrogen atom or a linear or branched C₁-C₆ alkyl radical, preferably methyl or ethyl; and
- Y⁻ denotes an anion derived from a mineral or organic acid or a halide, preferably bromide, chloride, acetate, borate, citrate, tartrate, bisulfate, bisulfite, methosulfate, sulfate or phosphate anion.

8. Composition according to any one of the preceding claims, wherein the polymer(s) comprising one or more cationic or quaternized acrylamide and/or methacrylamide unit(s) is(are) chosen from amphoteric polymers, and preferably from amphoteric polymers comprising a repetition of:
(i) one or more units derived from a monomer of (meth)acrylamide type,
(ii) one or more units derived from a monomer of (meth)acrylamidoalkyltrialkylammonium type, and
(iii) one or more units derived from an acidic monomer of (meth)acrylic acid type.

9. Composition according to any one of the preceding claims, wherein the polymer(s) comprising one or more cationic or quaternized acrylamide and/or methacrylamide unit(s) is(are) chosen from
- (meth)acrylamido(C₁-C₆ alkyl)tri(C₁-C₄ alkyl) ammonium halide/ (meth)acrylamide copolymers, preferably (meth)acrylamidopropyltrimonium chloride/ (meth)acrylamide copolymers, and more preferably acrylamidopropyltrimonium chloride/ acrylamide copolymers,
- (meth)acrylamido(C₁-C₆ alkyl)tri(C₁-C₄ alkyl) ammonium halide /(meth)acrylamide/(meth)acrylic acid terpolymers, preferably (meth)acrylamidopropyltrimonium chloride / (meth)acrylamide / (meth)acrylic acid terpolymers, more preferably acrylamide/ methacrylamidopropyltrimethylammonium chloride/ acrylic acid terpolymers,
- (meth)acrylamido(C₁-C₆ alkyl)tri(C₁-C₄ alkyl) ammonium halide/ (C₁-C₆ alkyl) (meth)acrylate / (meth)acrylic acid terpolymers, preferably (meth)acrylamidopropyltrimonium chloride / (C₁-C₆ alkyl) (meth)acrylate / (meth)acrylic acid terpolymers; more preferably acrylic acid/ methylacrylamidopropyltrimethylammonium chloride/ methyl acrylates terpolymers,
- and mixtures thereof.

10. Composition according to any one of the preceding claims, wherein the total amount of polymer(s) b) comprising one or more cationic or quaternized (meth)acrylamide units ranges from 0.01 to 5% by weight, preferentially from 0.015 to 4% by weight, more preferentially from 0.02 to 2% by weight, better from 0.04 to 1% by weight, and even better from 0.05 to 0.5% by weight, relative to the total weight of the composition.

11. Composition according to any one of the preceding claims, wherein the cationic polysaccharide(s) c) is(are) chosen from cationic celluloses, cationic galactomannan gums, and mixtures thereof; preferably from cellulose ether derivatives comprising quaternary ammonium groups, guar gums comprising cationic trialkylammonium groups, and mixtures thereof.

12. Composition according to any one of the preceding claims, wherein the total amount of cationic polysaccharide(s) c) ranges from 0.01 to 5% by weight, preferentially from 0.015 to 4% by weight, more preferentially from 0.02 to 3% by weight, better from 0.05 to 2% by weight, and even better from 0.1 to 1% by weight, relative to the total weight of the composition.

13. Composition according to any one of the preceding claims, **characterized in that** it further comprises one or more anionic surfactants; preferentially chosen from anionic surfactants of alkyl(ether) sulfate type; more preferentially from C₁₂-C₁₄ alkyl(ether) sulfate salts; even better from lauryl ether sulfate salts.

14. Composition according to any one of the preceding claims, **characterized in that** it further comprises one or more amphoteric or zwitterionic surfactants; preferentially chosen from (C₈-C₂₀ alkyl)betaines such as cocoylbetaine, (C₈-C₂₀ alkyl)amido(C₂-C₈ alkyl)betaines such as cocoylamido-propylbetaine, and mixtures thereof.

15. Process for treating keratin fibres, preferably for washing and/or conditioning keratin fibres, comprising the application onto the keratin fibres of a composition according to any one of the preceding claims.

## Patentansprüche

1. Zusammensetzung, umfassend:
a) eine Öl-in-Wasser-Emulsion, die eine D50-Teilchengröße von weniger als 350 nm aufweist, wobei die Teilchengröße unter Verwendung einer Vorrichtung Zetasizer von Malvern, Modell Nano-ZS, das auf der Photonenkorrelationsspektroskopie(PKS)-Methode basiert, gemessen wird, und Folgendes umfasst:
- eine Silikonmischung, umfassend (i) ein trialkylsilylterminiertes Dialkylpolysiloxan mit einer Viskosität von 40.000 bis weniger als 100.000 mPa.s bei 25 °C, wobei die Viskosität bei 25 °C unter Verwendung eines Anton-Paar-Rheometers; Modell MCR101, 25-6-Kegel; wobei die Einstellung "Zero Gap" vorgenommen wird, und mit einer Scherrate von 1 s⁻¹ für 2 Minuten gemessen wird, und (ii) ein Aminosilikon mit einer Viskosität von 1000 bis 15.000 mPa.s bei 25 °C, wobei die Viskosität bei 25 °C unter Verwendung eines Anton-Paar-Rheometers; Modell MCR101, Geometrie Einzelspaltzylinder: CC27-Spindel und einer Scherrate von 1 s⁻¹ für 2 Minuten gemessen wird, und einer Aminzahl von 2 bis 10 mg KOH pro Gramm Aminosilikon,
- eine Mischung von Emulgatoren, die einen oder mehrere nichtionische Emulgatoren umfasst, wobei die Mischung von Emulgatoren einen HLB-Wert von 10 bis 16 aufweist, und
- Wasser;
b) ein oder mehrere Polymere mit einer oder mehreren kationischen oder quaternisierten Acrylamid- und/oder Methacrylamid-Einheiten; und
c) ein oder mehrere kationische Polysaccharide, die von Polymeren b) verschieden sind;
wobei das Gewichtsverhältnis der Gesamtmenge des kationischen Polysaccharids bzw. der kationischen Polysaccharide c) zur Gesamtmenge des Polymers bzw. der Polymere b) mit einer oder mehreren kationischen oder quaternisierten Acrylamid- und/oder Methacrylamid-Einheiten im Bereich von 4 bis 20 liegt.

2. Zusammensetzung nach Anspruch 1, wobei das Gewichtsverhältnis der Gesamtmenge der kationischen Polysaccharide c) zur Gesamtmenge der Polymere b) mit einer oder mehreren kationischen oder quaternisierten Acrylamid- und/oder Methacrylamid-Einheiten im Bereich von 4 bis 10 liegt.

3. Zusammensetzung nach einem der vorhergehenden Ansprüche, wobei das trialkylsilylterminierte Dialkylpolysiloxan (i) die Formel (IX) aufweist:
R'₃SiO(R'₂SiO)ₚSiR'₃
wobei:
- R' gleich oder verschieden ist und für einen einwertigen Kohlenwasserstoffrest mit 1 bis 18 Kohlenstoffatomen, vorzugsweise 1 bis 6 Kohlenstoffatomen, steht und
- p für eine ganze Zahl von 500 bis 2000, vorzugsweise von 1000 bis 2000, steht; und vorzugsweise trimethylsilylterminiertes PDMS ist.

4. Zusammensetzung nach einem der vorhergehenden Ansprüche, wobei das Aminosilikon (ii) die Formel (X) aufweist:
XR₂Si(OSiAR)ₙ(OSiR₂)ₘOSiR₂X
wobei:
- R gleich oder verschieden ist und für einen einwertigen Kohlenwasserstoffrest mit 1 bis 18 Kohlenstoffatomen, vorzugsweise 1 bis 6 Kohlenstoffatomen, steht,
- X gleich oder verschieden ist und für R oder eine Hydroxylgruppe (OH) oder eine C₁-C₆-Alkoxygruppe steht; vorzugsweise X für R steht,
- A für einen Aminorest der Formel -R¹-[NR²-R³-]_{X}NR²₂ oder die protonierten Aminoformen des Aminorests steht, wobei R¹ für einen C₁-C₆-Alkylenrest, vorzugsweise einen Rest der Formel -CH₂CH₂CH₂- oder -CH₂CH(CH₃)CH₂- steht, R² gleich oder verschieden ist und für ein Wasserstoffatom oder einen C₁-C₄-Alkylrest, vorzugsweise ein Wasserstoffatom , steht, R³ für einen C₁-C₆-Alkylenrest, vorzugsweise einen Rest der Formel -CH₂CH₂-, steht und x für 0 oder 1 steht; und
- m+n für eine ganze Zahl von 50 bis 1000, vorzugsweise von 50 bis 600 steht; vorzugsweise A für einen Aminorest der Formel -R¹-[NR²-R³-]_{X}NR²₂ oder die protonierten Aminoformen des Aminorests, steht, wobei R¹ für -CH₂CH₂CH₂- oder -CH₂CH(CH₃)CH₂- steht, die Gruppen R² für Wasserstoffatome stehen, R³ für -CH₂CH₂- steht und x für 1 steht.

5. Zusammensetzung nach einem der vorhergehenden Ansprüche, wobei die Mischung von Emulgatoren einen oder mehrere nichtionische Emulgatoren umfasst, die ausgewählt sind aus:
(i) Polyoxyalkylenalkylethern, insbesondere (poly)ethoxylierten Fettalkoholen der Formel: R₃-(OCH₂CH₂)_{c}OH, wobei:
- R₃ für eine lineare oder verzweigte C₈-C₄₀-Alkyl- oder -Alkenylgruppe, vorzugsweise C₈-C₃₀-Alkyl- oder - Alkenylgruppe, die gegebenenfalls durch eine oder mehrere Hydroxylgruppen substituiert ist, steht und
- c für eine ganze Zahl zwischen 1 und 200 inklusive, vorzugsweise zwischen 2 und 150, steht;
und spezieller Fettalkoholen mit 8 bis 22 Kohlenstoffatomen, die mit 1 bis 30 mol Ethylenoxid oxethyliert sind (1 bis 30 OE);
(ii) Polyoxyalkylen(C₈-C₃₂)alkylphenylethern;
(iii) Polyoxyalkylensorbitan(C₈-C₃₂)fettsäureestern, speziell polyethoxylierten Fettsäureestern von Sorbitan, die vorzugsweise 2 bis 40 Ethylenoxideinheiten, weiter bevorzugt 2 bis 20 Ethylenoxideinheiten, enthalten; vorzugsweise polyoxyethylenierten Sorbitan(C₁₀-C₂₄)fettsäureestern, die vorzugsweise 2 bis 40 Ethylenoxideinheiten, ganz besonders bevorzugt 2 bis 20 Ethylenoxideinheiten, enthalten; und
(iv) polyoxyethylenierten (C₈-C₃₂)Fettsäureestern, die beispielsweise 2 bis 150 mol Ethylenoxid enthalten; vorzugsweise polyoxyethylenierten (C₁₀-C₂₄)Fettsäureestern, die beispielsweise 2 bis 150 mol Ethylenoxid enthalten.

6. Zusammensetzung nach einem der vorhergehenden Ansprüche, wobei die Öl-in-Wasser-Emulsion a) eine D50-Teilchengröße von 100 bis 300 nm, vorzugsweise von 150 bis 250 nm, weiter bevorzugt von 150 bis 225 nm und noch weiter bevorzugt von 160 bis 200 nm, ausgedrückt in Volumen, aufweist.

7. Zusammensetzung nach einem der vorhergehenden Ansprüche, wobei das Polymer bzw. die Polymere mit einer oder mehreren kationischen oder quaternisierten Acrylamid- und/oder Methacrylamid-Einheiten aus Homopolymeren oder Copolymeren mit mindestens einer der Einheiten der folgenden Formeln ausgewählt ist bzw. sind: 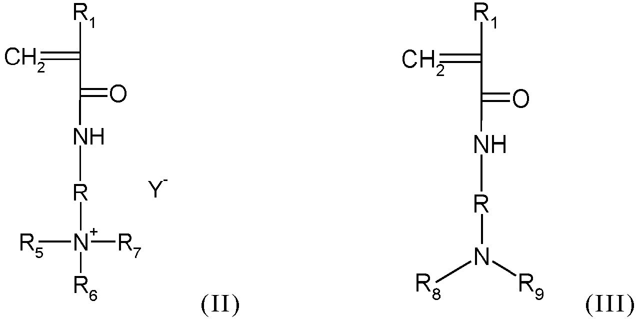 in denen:
- die Gruppen R₁, die gleich oder verschieden sein können, ein Wasserstoffatom oder einen CH₃-Rest bedeuten;
- die Gruppen R, die gleich oder verschieden sein können, einen linearen oder verzweigten C₁-C₁₂-Alkylrest, vorzugsweise einen linearen C₁-C₆-Alkylrest, der gegebenenfalls durch einen oder mehrere Hydroxylreste substituiert ist, bedeuten;
- R₅, R₆ und R₇, die gleich oder verschieden sein können, einen linearen oder verzweigten C₁-C₁₈-Alkylrest oder einen Benzylrest, vorzugsweise einen linearen oder verzweigten C₁-C₆-Alkylrest, bedeuten;
- R₈ und R₉, die gleich oder verschieden sein können, ein Wasserstoffatom oder einen linearen oder verzweigten C₁-C₆-Alkylrest, vorzugsweise Methyl oder Ethyl, bedeuten; und
- Y⁻ ein Anion bedeutet, das sich von einer mineralischen oder organischen Säure oder einem Halogenid ableitet, vorzugsweise ein Bromid-, Chlorid-, Acetat-, Borat-, Citrat-, Tartrat-, Bisulfat-, Bisulfit-, Methosulfat-, Sulfat- oder Phosphatanion.

8. Zusammensetzung nach einem der vorhergehenden Ansprüche, wobei das Polymer bzw. die Polymere mit einer oder mehreren kationischen oder quaternisierten Acrylamid- und/oder Methacrylamid-Einheiten ausgewählt ist bzw. sind aus amphoteren Polymeren und vorzugsweise aus amphoteren Polymeren, umfassend eine Wiederholung von:
(i) einer oder mehreren Einheiten, die sich von einem Monomer vom (Meth)acrylamid-Typ ableiten,
(ii) einer oder mehreren Einheiten, die sich von einem Monomer vom (Meth)acrylamidoalkyltrialkylammonium-Typ ableiten, und
(iii) einer oder mehreren Einheiten, die sich von einem sauren Monomer vom (Meth)acrylsäure-Typ ableiten.

9. Zusammensetzung nach einem der vorhergehenden Ansprüche, wobei das Polymer bzw. die Polymere mit einer oder mehreren kationischen oder quaternisierten Acrylamid- und/oder Methacrylamid-Einheiten ausgewählt ist bzw. sind aus:
- (Meth)acrylamido(C₁-C₆-alkyl)tri(C₁-C₄-alkyl)ammoniumhalogenid/(Meth)acrylamid-Copolymeren, vorzugsweise (Meth)acrylamidopropyltrimoniumchlorid/(Meth)acrylamid-Copolymeren und weiter bevorzugt Acrylamidopropyltrimoniumchlorid/Acrylamid-Copolymeren,
- (Meth)acrylamido(C₁-C₆-alkyl)tri(C₁-C₄-alkyl)ammoniumhalogenid/(Meth)acrylamid/(Meth)acrylsäur e-Terpolymeren, vorzugsweise (Meth)acrylamidopropyltrimoniumchlorid/(Meth)acrylamid/ (Meth)acrylsäure-Terpolymeren, weiter bevorzugt Acrylamid/Methacrylamidopropyltrimethylammoniumchlorid/ Acrylsäure-Terpolymeren,
- (Meth)acrylamido(C₁-C₆-alkyl)tri(C₁-C₄-alkyl)ammoniumhalogenid/(C₁-C₆-Alkyl)(meth)acrylat/(Meth)acrylsäure-Terpolymeren, vorzugsweise (Meth)acrylamidopropyltrimoniumchlorid/(C₁-C₆-Alkyl)(meth)acrylat/(Meth)acrylsäure-Terpolymeren; weiter bevorzugt Acrylsäure/Methylacrylamidopropyltrimethylammoniumchlor id/Methylacrylat-Terpolymeren,
- und Mischungen davon.

10. Zusammensetzung nach einem der vorhergehenden Ansprüche, wobei die Gesamtmenge an Polymer bzw. Polymeren b) mit einer oder mehreren kationischen oder quaternisierten (Meth)acrylamid-Einheiten im Bereich von 0,01 bis 5 Gew.-%, vorzugsweise von 0,015 bis 4 Gew.-%, weiter bevorzugt von 0,02 bis 2 Gew.-%, besser von 0,04 bis 1 Gew.-% und noch besser von 0,05 bis 0,5 Gew.-%, bezogen auf das Gesamtgewicht der Zusammensetzung, beträgt.

11. Zusammensetzung nach einem der vorhergehenden Ansprüche, wobei das kationische Polysaccharid bzw. die kationischen Polysaccharide c) aus kationischen Cellulosen, kationischen Galactomannangummen und Mischungen davon, vorzugsweise aus Celluloseetherderivaten mit quartären Ammoniumgruppen, Guargummen mit kationischen Trialkylammoniumgruppen und Mischungen davon ausgewählt ist bzw. sind.

12. Zusammensetzung nach einem der vorhergehenden Ansprüche, wobei die Gesamtmenge an kationischem Polysaccharid bzw. kationischen Polysacchariden c) im Bereich von 0,01 bis 5 Gew.-%, vorzugsweise von 0,015 bis 4 Gew.-%, weiter bevorzugt von 0,02 bis 3 Gew.-%, besser von 0,05 bis 2 Gew.-% und noch besser von 0,1 bis 1 Gew.-%, bezogen auf das Gesamtgewicht der Zusammensetzung, beträgt.

13. Zusammensetzung nach einem der vorhergehenden Ansprüche, **dadurch gekennzeichnet, dass** sie ferner ein oder mehrere anionische Tenside umfasst, die vorzugsweise aus anionischen Tensiden vom Alkyl(ether)sulfat-Typ, weiter bevorzugt aus C₁₂-C₁₄-Alkyl(ether)sulfat-Salzen, noch besser aus Laurylethersulfat-Salzen, ausgewählt sind.

14. Zusammensetzung nach einem der vorhergehenden Ansprüche, **dadurch gekennzeichnet, dass** sie ferner ein oder mehrere amphotere oder zwitterionische Tenside umfasst, die vorzugsweise aus (C₈-C₂₀-Alkyl)betainen wie Cocoylbetain, (C₈-C₂₀-Alkyl)amido(C₂-C₈-alkyl)betainen wie Cocoylamidopropylbetain und Mischungen davon ausgewählt sind.

15. Verfahren zur Behandlung von Keratinfasern, vorzugsweise zum Waschen und/oder Konditionieren von Keratinfasern, umfassend das Aufbringen einer Zusammensetzung nach einem der vorhergehenden Ansprüche auf die Keratinfasern.

## Revendications

1. Composition comprenant :
a) une émulsion huile-dans-eau ayant une taille de particule D50 inférieure à 350 nm, la taille de particule étant mesurée en utilisant un dispositif ZetaSizer de Malvern, modèle Nano-ZS qui est basé sur le procédé de spectroscopie par corrélation photonique (PCS), et comprenant :
- un mélange de silicones comprenant (i) un dialkylpolysiloxane à terminaisons trialkylsilyle ayant une viscosité de 40 000 à moins de 100 000 mPa.s à 25 °C, la viscosité étant mesurée à 25 °C à l'aide d'un rhéomètre Anton Paar ; modèle MCR101, cône 25-6 ; la configuration « interstice nul » étant réalisée et avec vitesse de cisaillement de 1 s⁻¹ pendant 2 minutes, et
(ii) une silicone aminée ayant une viscosité de 1 000 à 15 000 mPa.s à 25 °C, la viscosité étant mesurée à 25 °C à l'aide d'un rhéomètre Anton Paar ; modèle MCR101, géométrie cylindre à interstice unique : tige CC27 et vitesse de cisaillement de 1 s⁻¹ pendant 2 minutes, et un indice d'amine de 2 à 10 mg de KOH par gramme de silicone aminée,
- un mélange d'émulsifiants comprenant un ou plusieurs émulsifiants non-ioniques, dans laquelle le mélange d'émulsifiants présente une valeur de HLB de 10 à 16, et
- de l'eau ;
b) un ou plusieurs polymères comprenant une ou plusieurs unités acrylamide et/ou méthacrylamide cationiques ou quaternisés ; et
c) un ou plusieurs polysaccharides cationiques différents des polymères b) ;
dans laquelle le rapport pondéral de la quantité totale dudit ou desdits polysaccharides cationiques c) sur la quantité totale dudit ou desdits polymères b) comprenant une ou plusieurs unités acrylamide et/ou méthacrylamide cationiques ou quaternisés se situe dans la gamme allant de 4 à 20.

2. Composition selon la revendication 1 dans laquelle le rapport pondéral de la quantité totale desdits polysaccharides cationiques c) sur la quantité totale desdits polymères b) comprenant une ou plusieurs unités acrylamide et/ou méthacrylamide cationiques ou quaternisés se situe dans la gamme allant de 4 à 10.

3. Composition selon l'une quelconque des revendications précédentes, dans laquelle le dialkylpolysiloxane à terminaisons trialkylsilyle (i) est de formule (IX) :
R'₃SiO(R'₂SiO)ₚSiR'₃
dans laquelle :
- R', identique ou différent, est un radical hydrocarboné monovalent ayant de 1 à 18 atomes de carbone, de préférence de 1 à 6 atomes de carbone, et
- p est un entier de 500 à 2 000, de préférence de 1 000 à 2 000 ; et de préférence est un PDMS à terminaison triméthylsilyle.

4. Composition selon l'une quelconque des revendications précédentes dans laquelle la silicone aminée (ii) est de formule (X) :
XR₂Si(OSiAR)ₙ(OSiR₂)ₘOSiR₂X
dans laquelle :
- R, identique ou différent, est un radical hydrocarboné monovalent ayant de 1 à 18 atomes de carbone, de préférence de 1 à 6 atomes de carbone,
- X, identique ou différent, est R ou un hydroxyle (OH) ou un groupe C₁-C₆-alcoxy ; de préférence X est R,
- A est un radical amine de formule -R¹-[NR²-R³-]_{X}NR²₂, ou les formes amine protonées dudit radical amine, où R¹ est un radical C₁-C₆-alkylène, de préférence un radical de formule -CH₂CH₂CH₂- ou -CH₂CH(CH₃)CH₂-, R², identique ou différent, est un atome d'hydrogène ou un radical alkyle en C₁-C₄, de préférence un atome d'hydrogène, R³ est un radical C₁-C₆-alkylène, de préférence un radical de la formule -CH₂CH₂-, et x est 0 ou 1 ; et
- m+n est un entier de 50 à 1 000, de préférence de 50 à 600 :
de préférence A est un radical amine de formule -R¹-[NR²-R³-]_{X}NR²₂, ou les formes amine protonées dudit radical amine, dans laquelle R¹ est -CH₂CH₂CH₂- ou -CH₂CH(CH₃)CH₂-, R² sont des atomes d'hydrogène, R³ est -CH₂CH₂- et x est 1.

5. Composition selon l'une quelconque des revendications précédentes, dans laquelle le mélange d'émulsifiants comprend un ou plusieurs émulsifiants non-ioniques choisis parmi :
(i) les éthers d'alkyle de polyoxyalkylène, notamment les alcools gras (poly)éthoxylés de formule : R₃-(OCH₂CH₂)_{c}OH où :
- R₃ représente un groupe alkyle ou alcényle en C₈-C₄₀, linéaire ou ramifié, de préférence en C₈-C₃₀, éventuellement substitué par un ou plusieurs groupes hydroxy, et
- c est un entier compris inclusivement entre 1 et 200, préférentiellement entre 2 et 150 ;
et plus particulièrement les alcools gras comprenant de 8 à 22 atomes de carbone, oxyéthylénés par 1 à 30 moles d'oxyde d'éthylène (1 à 30 OE) ;
(ii) les éthers de (C₈-C₃₂)alkylphényle de polyoxyalkylène ;
(iii) les esters d'acide gras (C₈-C₃₂) de polyoxyalkylène sorbitane, notamment les esters d'acide gras polyéthoxylés de sorbitane contenant de préférence de 2 à 40 motifs d'oxyde d'éthylène, plus préférentiellement de 2 à 20 motifs d'oxyde d'éthylène ; de préférence les esters d'acide gras (C₁₀-C₂₄) de sorbitane polyoxyéthyléné ayant de préférence de 2 à 40 motifs d'oxyde d'éthylène, plus préférentiellement de 2 à 20 motifs d'oxyde d'éthylène ; et ;
(iv) les esters d'acide gras (C₈-C₃₂) polyoxyéthylénés contenant par exemple de 2 à 150 moles d'oxyde d'éthylène ; de préférence les esters d'acide gras (C₁₀-C₂₄) polyoxyéthylénés ayant par exemple de 2 à 150 moles d'oxyde d'éthylène.

6. Composition selon l'une quelconque des revendications précédentes, dans laquelle l'émulsion huile-dans-eau a) présente une taille de particule D50 de 100 à 300 nm, de préférence de 150 à 250 nm, de préférence encore de 150 à 225 nm, et encore plus préférentiellement de 160 à 200 nm, exprimée en volume.

7. Composition selon l'une quelconque des revendications précédentes, dans laquelle le ou les polymères comprenant une ou plusieurs unités acrylamide et/ou méthacrylamide cationiques ou quaternisés sont choisis parmi les homopolymères ou copolymères comprenant au moins l'une des unités de formules suivantes : 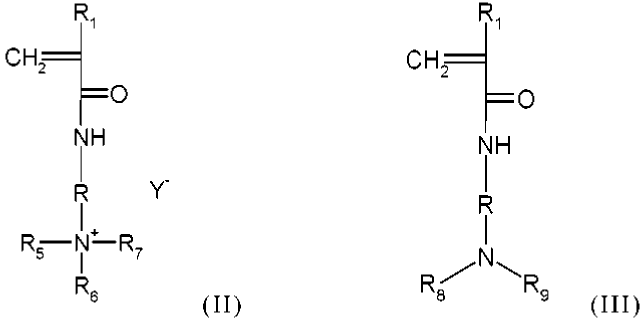 dans lesquelles :
- R₁, identiques ou différents, désignent un atome d'hydrogène ou un radical CH₃ ;
- R, identiques ou différents, désignent un radical alkyle en C₁-C₁₂ linéaire ou ramifié, de préférence un radical alkyle en C₁-C₆ linéaire, éventuellement substitué par un ou plusieurs radicaux hydroxy ;
- R₅, R₆ et R₇, identiques ou différents, désignent un radical alkyle en C₁-C₁₈, linéaire ou ramifié, ou un radical benzyle, de préférence un radical alkyle en C₁-C₆, linéaire ou ramifié ;
- R₈ et R₉, identiques ou différents, désignent un atome d'hydrogène ou un radical alkyle en C₁-C₆, linéaire ou ramifié, de préférence méthyle ou éthyle ; et
- Y⁻ désigne un anion dérivé d'un acide minéral ou organique ou un anion halogénure, de préférence bromure, chlorure, acétate, borate, citrate, tartrate, bisulfate, bisulfite, méthosulfate, sulfate ou phosphate.

8. Composition selon l'une quelconque des revendications précédentes, dans laquelle le ou les polymères comprenant une ou plusieurs unités acrylamide et/ou méthacrylamide cationiques ou quaternisés sont choisis parmi des polymères amphotères, et de préférence parmi les polymères amphotères comprenant une répétition de :
(i) une ou plusieurs unités issues d'un monomère de type (méth)acrylamide,
(ii) une ou plusieurs unités issues d'un monomère de type (méth)acrylamidoalkyltrialkylammonium, et
(iii) une ou plusieurs unités issues d'un monomère acide de type acide (méth)acrylique.

9. Composition selon l'une quelconque des revendications précédentes, dans laquelle le ou les polymères comprenant une ou plusieurs unités acrylamide et/ou méthacrylamide cationiques ou quaternisés sont choisis parmi
- les copolymères halogénure de (méth)acrylamido(alkyle en C₁-C₆)-tri(alkyle en C₁-C₄)ammonium/(méth)acrylamide, de préférence les copolymères chlorure de (méth)acrylamidopropyltrimonium/(méth)acrylamide, et encore plus préférentiellement les copolymères de chlorure d'acrylamidopropyltrimonium/acrylamide,
- les terpolymères (méth)acrylamido(alkyle en C₁-C₆)-tri(alkyle en C₁-C₄)ammonium/(méth)acrylamide/acide (méth)acrylique, de préférence les terpolymères chlorure de (méth)acrylamidopropyltrimonium/(méth)acrylamide/acide (méth)acrylique, plus préférentiellement les terpolymères acrylamide/chlorure de méthacrylamidopropyltriméthylammonium/acide acrylique,
- les terpolymères halogénure de (méth)acrylamido(alkyle en C₁-C₆)-tri(alkyle en C₁-C₄)ammonium/(méth)acrylate de (alkyle en C₁-C₆)/acide (méth)acrylique, de préférence les terpolymères chlorure de (méth)acrylamidopropyltrimonium/(méth)acrylate de (alkyle en C₁-C₆)/acide (méth)acrylique ; plus préférentiellement les terpolymères acide acrylique/chlorure de méthylacrylamidopropyltriméthylammonium/acrylates de méthyle,
- et leurs mélanges.

10. Composition selon l'une quelconque des revendications précédentes, dans laquelle la teneur totale du ou des polymères b) comprenant une ou plusieurs unités de (méth)acrylamide cationiques ou quaternisés se situe dans la gamme allant de 0,01 % à 5 % en poids, de préférence de 0,015 % à 4 % en poids, plus préférentiellement de 0,02 % à 2 % en poids, mieux encore de 0,04 % à 1 % en poids, toujours mieux de 0,05 % à 0,5 % en poids, par rapport au poids total de la composition.

11. Composition selon l'une quelconque des revendications précédentes, dans laquelle le ou les polysaccharides cationiques c) est(sont) choisis parmi les celluloses cationiques, les gommes de galactomannane cationiques, et leurs mélanges ; de préférence parmi les dérivés d'éther de cellulose comportant des groupements ammonium quaternaire, les gommes de guar comprenant des groupements trialkylammonium cationiques et leurs mélanges.

12. Composition selon l'une quelconque des revendications précédentes, dans laquelle la quantité totale du ou des polysaccharides cationiques c) se situe dans la gamme allant de 0,01 % à 5 % en poids, de préférence de 0,015 % à 4 % en poids, plus préférentiellement de 0,02 % à 3 % en poids, mieux encore de 0,05 % à 2 % en poids, toujours mieux de 0,1 % à 1 % en poids, par rapport au poids total de la composition.

13. Composition selon l'une quelconque des revendications précédentes, **caractérisée en ce qu'**elle comprend en outre un ou plusieurs tensioactifs anioniques ; préférentiellement choisis parmi les tensioactifs anioniques de type sulfate d'alkyl(éther) ; plus préférentiellement parmi les sels de sulfate d'alkyl(éther) en C₁₂-C₁₄ ; même encore mieux parmi les sels de sulfate de lauryléther.

14. Composition selon l'une quelconque des revendications précédentes, **caractérisée en ce qu'**elle comprend en outre un ou plusieurs tensioactifs amphotères ou zwittérioniques ; préférentiellement choisis parmi les (alkyle en C₈-C₂₀)bétaïnes telles que la cocoylbétaïne, les (alkyle C₈-C₂₀)amido(alkyle en C₂-C₈)bétaïnes telles que la cocoylamido-propylbétaïne et leurs mélanges.

15. Procédé de traitement des fibres kératiniques, de préférence de lavage et/ou de conditionnement des fibres kératiniques, comprenant l'application sur les fibres kératiniques d'une composition selon l'une quelconque des revendications précédentes.
